(19)

Europäisches
Patentamt
European
Patent Office
Office européen
des brevets

(11) **EP 2 528 016 A1**

(12) **EUROPEAN PATENT APPLICATION**

(43) Date of publication:
**28.11.2012 Bulletin 2012/48**

(51) Int Cl.:
**G06K 9/00** *(2006.01)*          **A61B 5/11** *(2006.01)*

(21) Application number: **11167737.3**

(22) Date of filing: **26.05.2011**

| | |
|---|---|
| (84) Designated Contracting States:<br>**AL AT BE BG CH CY CZ DE DK EE ES FI FR GB**<br>**GR HR HU IE IS IT LI LT LU LV MC MK MT NL NO**<br>**PL PT RO RS SE SI SK SM TR**<br>Designated Extension States:<br>**BA ME**<br><br>(71) Applicant: **FUJITSU LIMITED**<br>**Kawasaki-shi,**<br>**Kanagawa 211-8588 (JP)** | (72) Inventor: **Abedi, Saied**<br>**Reading, Berkshire RG1 4LY (GB)**<br><br>(74) Representative: **Wilding, Frances Ward**<br>**Haseltine Lake LLP**<br>**Lincoln House, 5th Floor**<br>**300 High Holborn**<br>**London WC1V 7JH (GB)**<br><br><u>Remarks:</u><br>Amended claims in accordance with Rule 137(2)<br>EPC. |

(54)   **A method for analysing sensor data**

(57)    A method for analysing sensor data in the form
of parameters describing the condition of a subject the
method comprising processing the sensor data to provide, at each of a plurality of time instants, a collection
of two or more of the parameters, each of which parameters describes a different aspect of the subject's condition, the parameters together representing a state of the
subject, putting together successive collections to form
a sequence of states; and analysing the sequence to
create a state machine representing the states of the
subject detected over a period of time comprising the
plurality of time instants.

FIG. 1

EP 2 528 016 A1

**Description**

The present invention relates to the analysis of sensor data. The sensor data relates to the condition of a subject, preferably a human subject. The sensor data can for example represent human behaviour using suitable parameters.

Human behaviour monitoring has been subject of many recent research studies. In particular, behaviour monitoring of elderly patients has been considered. To guarantee that elderly patients will receive the medical attention they need, they currently may have to sacrifice their independence and personal social life by either living in nursing homes or spending a significant amount of their time in a specialised hospital for continuous monitoring. Not only does this reduce their independence but it also affects, in a negative way, their psychological status.

It is thus desirable to find a way of monitoring elderly patients and other subjects in a way that structures the information collected about the subject's condition, so that it can be easily interpreted by a medical professional and/or by the lay person.

According to a first aspect, invention embodiments provide a method for analysing sensor data in the form of parameters describing the condition of a subject, the method comprising:

processing the sensor data to provide, at each of a plurality of time instants, a collection of two or more of the parameters, each of which parameters describes a different aspect of the subject's condition, the parameters together representing a state of the subject;
putting together successive collections to form a sequence of states; and
analysing the sequence to create a state machine representing the states of the subject detected over a period of time comprising the plurality of time instants.

Invention embodiments can provide a way of putting together parameters over time, each parameter describing a different aspect of the subject's condition, into a model that is easy to interpret. Specifically, invention embodiments can provide a state machine (a human state machine for human subjects) which is a model composed of a finite number of states and preferably indicating the transitions between those states. Here, each state of the subject is made up of two or more parameters at a certain time instance.

The sensor data is processed to provide this collection of two or more the parameters which represents the state. The particular processing required depends on the sensor data provided and on the condition to be monitored, for example. The method is designed to be computer implemented and therefore the programming can determine this processing.

Preferably, processing comprises: grouping the sensor data into groups, each group including all the parameters describing a single aspect of the subject's condition and the parameters in each group being mutually exclusive so that the group provides a data stream of the parameters successively present over time, and sampling the data from at least two data streams at different time instants, to form a state represented by a collection of the parameters which are present at each time instant, each collection including the parameter from each data stream that is sampled at that time instant.

Advantageously therefore, one embodiment provides a number of groups, each group relating to a single aspect of the subject's condition. Preferably the parameters in each group are mutually exclusive so that only one parameter will be present at a given time. For example, a binarised system can be used. However, other possibilities fall within the scope of the invention. The grouping described can allow the state to be formed by sampling the data from at least two data strands. At any one time instant, only one parameter will be "switched on" and thus sampled.

A network of sensors can be provided to feed the sensor data into the human state machine method. Alternatively, individual sensors or more than one network of sensors may be provided. In some circumstances the sensors/ sensor data will be pre-selected in order to correspond to one or more diseases or medical conditions to be investigated. Alternatively, there may be a selection stage in which a selecter selects sensor data which is relevant to one or more diseases or medical conditions to be investigated before inputting the sensor data. Sensor data may also be inputted and pre-processed. The pre-processing may binarise any non-binary values of the sensor data and provide mutually exclusive binarised parameters for the processing. The optional selection step mentioned above may take place between inputting and pre-processing or before inputting.

The skilled person will appreciate that there is likely to be a wealth of different types of sensor data which is available. In one preferred embodiment, the aspects included within the collections vary to give two or more different collection types, the sequence of collections preferably alternating between two different collection types.

This variation allows some reduction in the amount of data fed through the method, and is effectively a type of selection. Different collection types can be tailored to the condition which is being monitored. For example, parameters of a certain more important group may always be included, whereas parameters of one of more other groups which are not so important may be present only at every second, third or fourth time instant.

The collection type can change more or less frequently depending on the implementation. Preferably, the collection type changes at each new time instant, or at every second time instant.

The aspects describing the subject's condition can be chosen according to the implementation. For example, the aspect may include any of whereabouts, activity, heart condition, blood oxygenation level and mobility.

Turning now to the analysis stage of the method, analysing the state machine may include storing all the states detected over the period of time and the transitions between them, and preferably includes calculation of the total time spent in each state and the probability of transition to other detected states.

The state machine referred to in the previous paragraphs contains only states which have been derived for the subject over a period of time. However, the skilled reader will appreciate that that particular state machine is only a part of a system model which includes the set of all the potential states which could be detected. The system model can be useful in processing.

Visualisation of the state machine is of tremendous value and one advantageous display (whether on a screen, on a print out or elsewhere) is based on a grid of potential states. For example, the grid may have N axes, an axis for each of N aspects displayed, the states detected being represented on the grid, transitions between states preferably also being represented. Alternatively, two or more aspects may be displayed consecutively along an axis, particularly if they are present in alternation in different collection types.

The potential states include all the states within the system model for the aspects being displayed. Thus the display may omit one or more aspects. For example if a collection contains three parameters, a 2D grid might be provided to show just those two parameters. More usually, an axis (or part of an axis) is provided for each aspect included within the collections. Transitions between the states may be represented using a line between two states, preferably with an arrow pointing in the direction of transition between the two states. Where there have been no transitions between two states there will be no such line. Alternatively or additionally, the state machine can be displayed along with the timing of the states. Here, the state machine is for example displayed based on a grid of potential states and time, with N+1 axes, an axis for each of N aspects displayed and an axis for time, the states detected and their timing being represented. Alternatively, and as before, more than one axis can be displayed along a single axis.

Once the state machine has been created, or as a general step for the system model, the states can be assessed to check whether they are desirable or not. For example, according to some preferred embodiments, each state detected in the state machine or each potential state of the system model is compared to a database of medical rules, and preferably wherein a warning is provided if the state does not conform to the rules.

The database of medical rules is related to symptoms of one or more diseases or medical conditions; and preferably each rule may correspond to at least one symptom of the disease or medical condition. The result of the comparison can be used for diagnostic purposes. The states can be classified as desirable or undesirable. In preferred embodiments, the result of the comparison is a classification of each state as in violation of, in line with, or not relevant to one or more of the rules, and each state is marked accordingly.

The marking can be a colour on a display of the state machine or of the system model. Preferably, the marking is provided on a representation of the states, with one marking for any state in violation of one or more of the rules, another marking for any state in line with one or more of the rules and a third marking for any state not relevant to one or more of the rules.

In one preferred embodiment, a method of identifying states in the system model or in the state machine allocates them to clusters according to their comparison with a database of rules.

In one alternative, the states in the system model are divided into a set of one or more clusters, including any of: a problem cluster for all the states which are in violation of one or more of the rules; a must-have cluster for all the states which are in line with one or more of the rules; and a don't care cluster for all the states which are not relevant to one or more of the rules; and each detected state is automatically allocated to a cluster of the system model.

This processing allows a system model to be provided in advance with information as to which states are suitable or unsuitable. Thus real time processing of detected states may be faster, may not require extra information from a database and may re-use system model clusters on several occasions.

In a second alternative, each detected state is compared with the database of medical rules and the detected states are divided into a set of one or more clusters, including any of: a problem cluster for all the states which are in violation of one or more of the rules; a must-have cluster for all the states which are in line with one or more of the rules; and a don't care cluster for all the states which are not relevant to one or more of the rules. This second alternative does not require processing of all the states in the system model but only clustering of the actual detected states.

In either case, a set of clusters may be provided for each rule of a grouping of rules corresponding to the symptoms of a disease or medical condition, and two or more sets of clusters may be combined to form a set of overall clusters taking into account two or more symptoms.

The states in each cluster can be distinguished by colour coding or any other suitable differentiation.

One of the uses of the human state machine method is in checking progress of a disease or medical condition over time. Thus, the sequence of state can be analysed over two or more periods of time to create a plurality of different state machine versions. The periods of time maybe consecutive or spaced.

According to a second aspect of the present invention, embodiments provide a state machine apparatus arranged to analyse sensor data in the form of parameters describing the condition of a subject, the apparatus comprising:

a processor operable to process the sensor data to provide, at each of a plurality of time instants, a collection of two or more of the parameters, each of which parameters describes a different aspect of the subject's condition, the parameters together representing a state of the subject;
a sequencer operable to put together successive collections to form a sequence of states; and
an analyser operable to analyse the sequence to create a state machine representing the states of the subject detected over a period of time comprising the plurality of time instants.

This apparatus aspect corresponds to the first method aspect and features of the method aspect can be applied also to the apparatus aspect. The skilled reader will appreciate that although the state machine apparatus is defined to include a processor sequencer and analyser, these different constituent parts may be all provided by the same basic computing capability. Equally, any storage, processing, and display functionality can be provided by the same computing apparatus.

All functional items described herein with respect to the computing apparatus may be embodied as one or more suitably programmed processors accessing memory and input/output devices as necessary. The same processor or processors may act as any or all of the functional items.

In a further aspect, invention embodiments provide a sensing system operable to analyse data in the form of parameters describing the condition of a subject. The system may comprise an apparatus according to the second aspect and one or more sensors which are operable to gather information which can be used to describe the condition of the subject.

In a final aspect, there may be provided a computer program which when executed on a computing apparatus causes it to carry out the method as described hereinbefore, or which when downloaded onto a computing apparatus causes it to become the state machine apparatus as it is currently known before.

Features and sub-features of any of the aspects all form part of the same general invention concept and may be freely combined unless clearly incompatible.

Finally, for the avoidance of doubt it is noted that invention embodiments also provide a computer program product for carrying out any of the methods described herein, and a computer readable medium having stored thereon a program for carrying out any of the methods described herein. A computer program embodying the invention may be stored on a computer-readable medium, or it could, for example, be in the form of a signal such as a downloadable data signal provided from an Internet website, or it could be in any other form.

Preferred features of the present invention will now be described, purely by way of example, with reference to the accompanying drawings, in which:-

Figure 1 is a flow chart related to the basic embodiment of invention;
Figure 2 is a block diagram showing the components of a basic embodiment of the present inventions;
Figure 3 is a schematic diagram showing the grouping and classification function to create three mainstreams of data of whereabouts, activities and gait;
Figure 4 is a schematic diagram showing the sampling function creating a sequences of two parameter states;
Figure 5 is a sampling function creating the two parameter state;
Figure 6 is a schematic diagram of a buffering and state machine formation function which creates the state machine;
Figure 7 is a schematic diagram of the overall architecture for creation of a human state machine based on raw sensor data;
Figure 8 is a schematic diagram of cluster creation;
Figure 9 is a specific example of a grouping and classification function;
Figure 10 is a specific example of a sampling function;
Figure 11 is a specific example of the sampling function creating the two parameter states;
Figure 12 is a specific example of the offering and state machine formation function;
Figure 13 shows the overall architecture for creation of the human state machine with a MATLAB implementation;
Figure 14 shows the outcome of the MATLAB platform for one week of test data for a normal person;
Figure 15 shows the outcome of the MATLAB platform for one week of test data for an Alzheimer's patient;
Figure 16 shows the outcome of the MATLAB platform for day 1 for a normal person;
Figure 17 shows the outcome of the MATLAB platform for day 8 for an Alzheimer's patient;
Figure 18 shows the outcome of the MATLAB platform for week one for a normal person;
Figure 19 shows the outcome of the MATLAB platform for week two for a person with Alzheimer's disease;
Figure 20 shows a basic grid of states for an overall system model;

Figure 21 shows the grid of states and classifications of the walking symptom;
Figure 22 shows the grid of states with the memory loss symptom and exercise based clusters;
Figure 23 shows the grid with memory and walking status;
Figure 24 is a 3D representation of the memory related rules for the human state machine;
Figure 25 is a schematic diagram of creation of clusters;
Figure 26 is a schematic diagram of cluster grouping;
Figure 27 shows a representation of a human state machine for day 1 together with a 3D representation thereof;
Figure 28 shows a representation of a human state machine for day 134 together with a 3D representation thereof;
Figure 29 shows a representation of a human state machine for day 162 together with a 3D representation thereof;
Figure 30 shows a representation of a human state machine for day 202 together with a 3D representation thereof;
Figure 31 shows a representation of a human state machine for day 206 together with a 3D representation thereof;
Figure 32 shows a human state machine for week 1;
Figure 33 shows a human state machine for week 17;
Figure 34 shows a human state machine for week 25; and
Figure 35 shows a human state machine for week 34.

Invention embodiments aim to propose an intelligent, seamless and preferably non-intrusive passive sensing network to improve the situation faced by the patients. Invention embodiments propose an architecture which establishes a human state machine for (behaviour) monitoring. The architecture can be flexible and can be applied to different diseases or generic healthcare monitoring. It can also be applied to behaviour monitoring for non-human subjects, such as animals, birds and other non-human life forms.

To achieve a cheap and affordable monitoring system, invention embodiments take the first step by establishing a novel architecture for extraction of a state machine out of 'raw' sensory data.

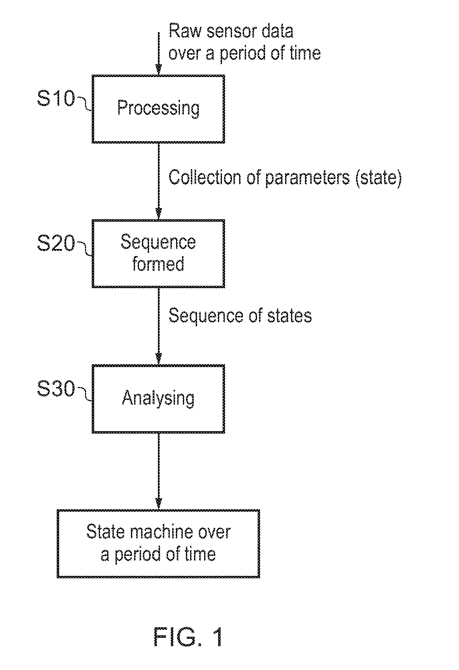

Figure 1 is a flow chart of a general embodiment of the invention. Raw sensor data arriving over a period of time is fed to processing in step S10. This processing forms a collection of parameters which together define a state. In the next step S20, a sequence of states is formed. The next step is analysing S30, which leads to the provision of a state machine over the period of time.

Some invention embodiments perform the following specific functional steps: selecting the raw sensor data based on their relevance to the symptoms of the disease of interest; and grouping and categorising those raw sensor data based on symptom related states to form a human state machine whereby a flexible N-dimensional grid of parameters can be formed based on the symptoms of any disease for example 2-dimensional activity-behaviour states.

Some invention embodiments then process the human state machine by comparing each state against predefined rules from a symptom-related rule database that indicates if the parameters embedded in the state of behaviour are in violation of the symptom-related rules, and then classifying them into the relevant clusters; then dividing all the states into ones that their parameters violate the symptom related rules, 'don't care' and the ones which do not violate the symptoms related rules; and then colour coding the clusters. By changing the sensor data (inputting new raw sensor data) it is possible to adapt the human state machine in a dynamic way.

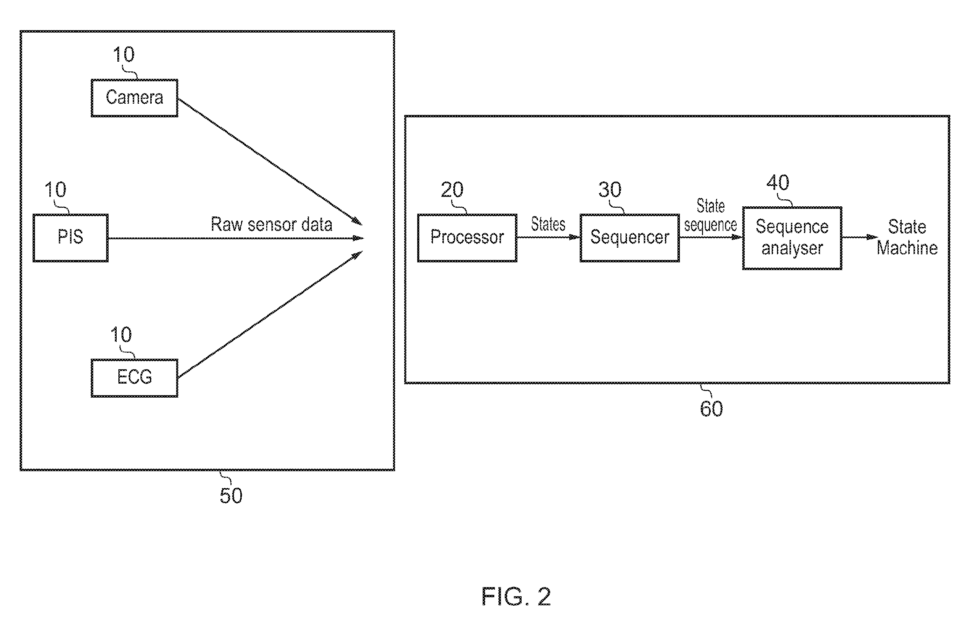

Figure 2 is a schematic diagram of hardware used to carry out the method. A sensor network is shown as block 50. Sensors include a camera, a passive infra-red sensor, and an ECG. Sensor block 50 feeds sensor data to an apparatus 60. The apparatus includes a processor 20 which provides the states, sequencer 30 which gives a sequence of states and sequence analyser 40 which provides the state machine.

The sensor block 50 uses sensors to gather information from the environment; the information is somehow tied to the disease symptoms. For example, if a disease has mobility related symptoms the sensor data can measure the mobility aspects or whereabouts of patients or speed of patient movements. Or for example if the disease symptom has anything to do with the heart condition if may also measure the ECG. The sensor data is preferably gathered in a non-intrusive way but it can also be gathered in an intrusive way. In preferred embodiments the sensor data then is digitised into binary 0 and 1 levels (for example if somebody fallen or not, or for example if somebody is in a room or not). Each sensor can provide consecutive sensed values which together form a strand of data. Parallel strands of sensor data from the different sensors are then passed to a grouping and classification function as discussed in the next section.

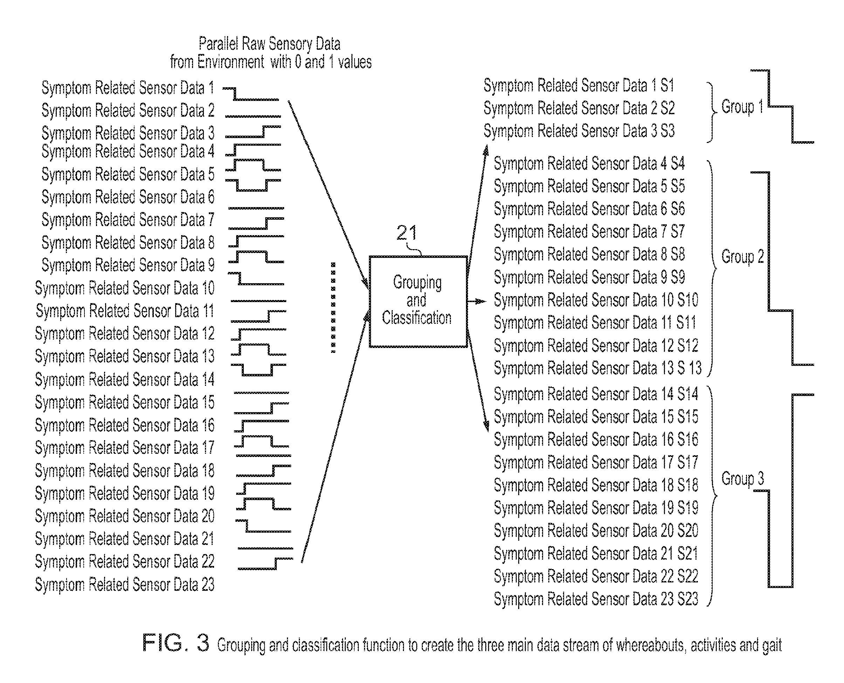

A grouping and classification function 21 may be carried out in processor 20 and used to group the sensor data (perhaps with values 0 and 1) into a number of main streams of data. For example, three groups of whereabouts, activities and gait may be provided as shown in Figure 3.

Figure 3 shows how the method takes 23 strands of data from the environment with 0 and 1 values and provides 3 groups of parameters each related to one aspect. Group 1 includes 3 data strands, each related to gait. Group 2 is a group of 10 data strands, each related to, for example, whereabouts and the final 10 strands of data are in group 3 which maybe related for example to activities.

The grouping and classification function may be a set of predefined rules to group the sensor binary data. For example if we are dealing with the walking state of the patient then one group can include three inputs of binary data:

S1: Walking normally (yes=1 no=0), S2 walking with difficulty (yes=1, no=0) or S3 fall while walking (yes=1, no=0) or if it is whereabouts, "Being Present in Kitchen" has two levels of (yes=1, zero=0).

The grouping and classification function transforms the input binary sensor data into a single decimal input wave form. Each level of this waveform represents a parameter of a state. We assume that only one of this phenomenon happens at each time (i.e having value=1). For example in the case of walking, discussed earlier, the three binary data sequences are converted into input data waveform with three possible levels of 1, 2 and 3. Consecutive values form a data stream of the parameter present over time.

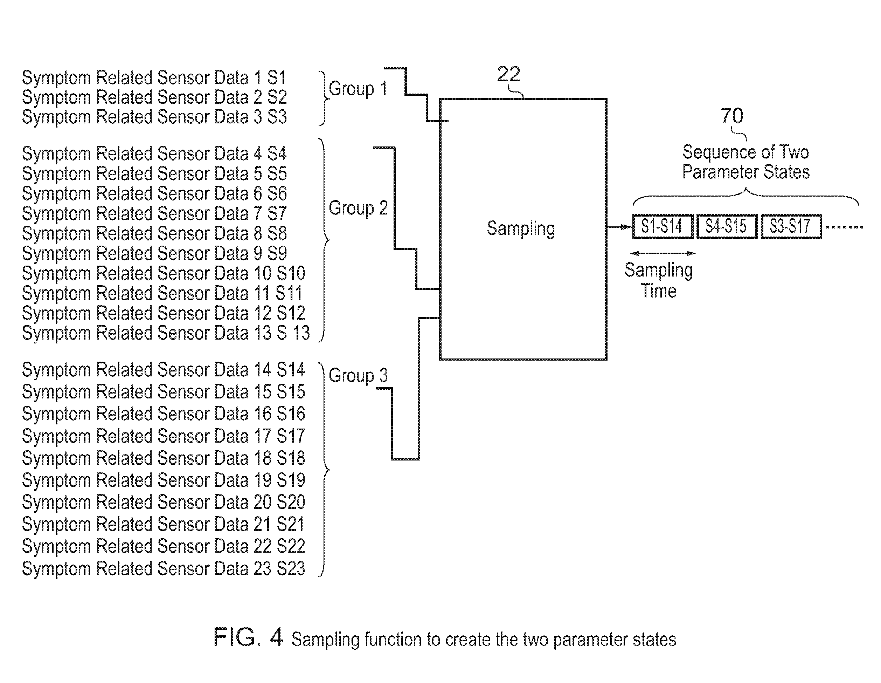

A sampling function 22 can also be carried out, for example in processor 20. The idea behind the sampling function is to create a state made up of two parameters (or more parameters) by sampling the data streams related to the groups of symptom related sensor data. In Figure 4 we have established a sequence of states of the groups of sensor data, each at a different sampling time. Figure 4 shows sampling to form a sequence of states based on which parameter in the group is present at a first, second and third time instant.

In Figure 4 for example, the order of the two parameters in any state may be activities and whereabouts. In this example, the activities are split into two groups, each group comprising mutually exclusive activities. For example S1, S4, S3 may refer to the gait or to the different activities of a patient at home and S14, S15 and S17 may be different whereabouts of the patient at home.

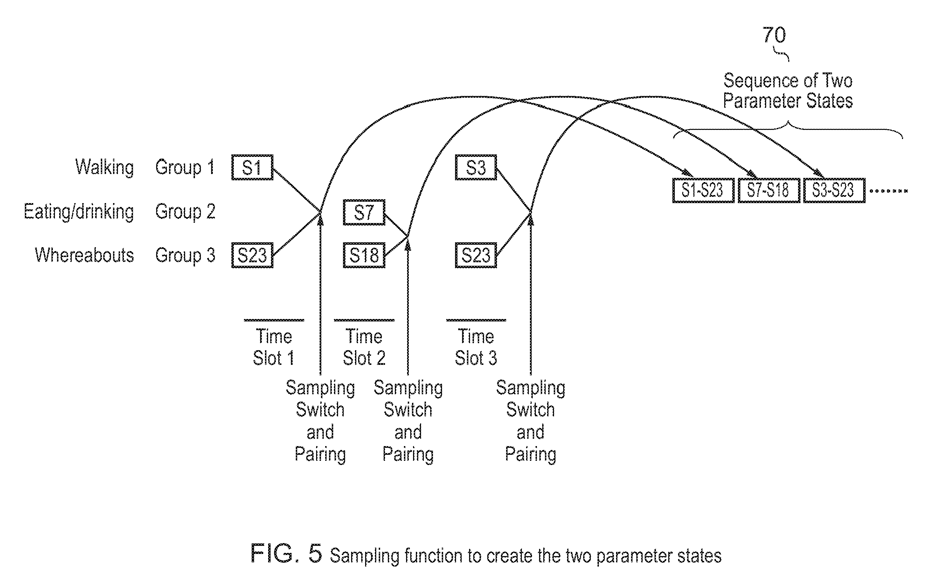

The idea behind the sampling function is explained in Figure 5 which demonstrates that at each point of time the sampling function takes a sample of different groups and forms the two-parameter states from the parameter present (that is with binary value '1') at that time instant in each group sampled. Sampling may rely on a simple database of rules which is able to indentify the nature of the incoming sensor data and put them into the relevant states.

Figure 5 is a pictorial description of how the sequence of two parameter states (70) can be provided. In the first time slot, the parameters from group 1 and group 3 are sampled to give S1 and S23. In the second time slot, groups 2 and 3 are sampled and the parameters present are S7 and S18. In a third time slot, group 1 and group 3 are sampled again and the parameters sampled are S1 and S23.

In Figure 5, the first parameter is switched between Group 1 (for example, activities related to walking quantity) and Group 2 (any other type of activities such as eating or drinking). The second parameter is from Group 3 (whereabouts). Alternatively, we may have a simple rule that the first state parameter is always formed based on the data from Group 3 (which may be the whereabouts), and the other parameter is picked by switching between the parameters related to two different group of activities: the Group 1 (for example activities related to walking quality) and the Group 2 (any other type of activities such as eating or drinking) parameters.

In a further alternative, one of the parameters may be heart rate or blood oxygenation level. There may be three or more parameters present in each state, depending on the circumstances and condition of subject.

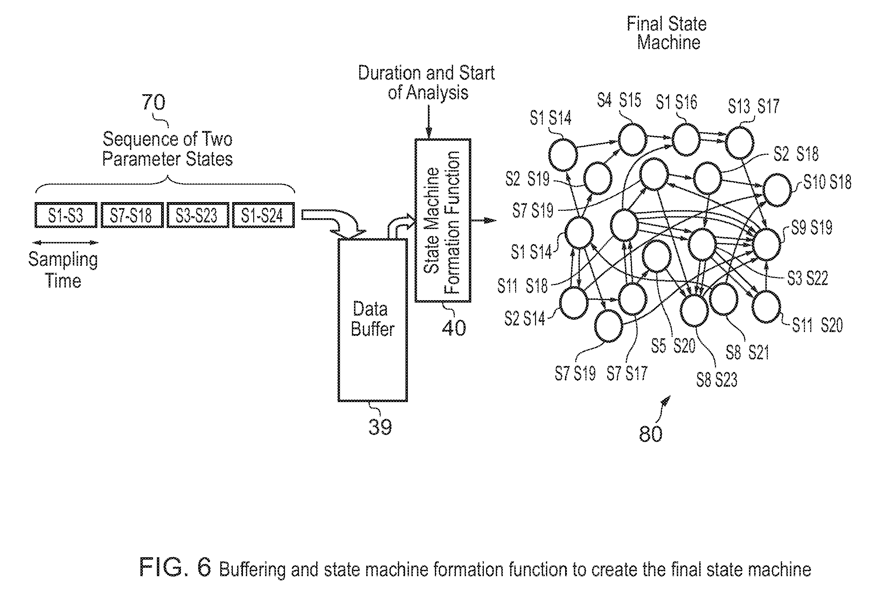

The sampling unit 22 may incorporate a sequencer which provides a sequence of states, one for each time slot/instant (also referred to as sampling time). The extracted sequence of the states may be buffered in buffer 39 for analysis. Based for example on the length of time and the starting point of the analysis, as input parameters, the state machine formation function scans through the buffered data and creates a state machine and determines its related probabilities. This is explained with reference to Figure 6.

Figure 6 shows a buffering process and state machine formation to create the state machine. This overview shows data fed to grouping and classification unit 21 and then sampled in sampler 22 to form a sequence of two parameter states as previously described. The sequence is fed to a data buffer for storage of the state over a period of time and then a state machine is created by state machine formation function 40, with the input of the duration and start of the analysis. The final state machine (80) is shown pictorially as a number of states in circles connected by the transitions between them. The arrow head indicates the direction of transition.

For the current state machine (corresponding to a time period from which the data is being processed) and for each state we calculate the ratio of the time spent to the total period (for example one month, one week or one day) so that:

$$Tr_{(s_k)} = \left( \sum_{i=1}^{I} Tspent_i \right) \Big/ T \quad , k = 1 \cdots K \qquad (1)$$

where $T$ is the total length of time for which the state machine is determined, $Tspent_i$ is the time spent in each occurrence of the state $k$ and $K$ is the total number of states.

For each state we calculate the total probability of transitions to other potential states as

$$\mathrm{Pr}_k = \sum_{d=1}^{D_k} \mathrm{Pr}\,obability(S_k \mapsto \{Sout_d\}), \quad k = 1, \cdots, K \qquad (2)$$

where $D_k$ is the number of states the state $S_k$ has transitions into. These values will be calculated and displayed for each state of the state machine. These values in (1) and (2) are important in determining how and when the transitions between different states of the human state machine happen. They are also useful in understanding the main habits or the states that are dominant from the time perspective. These metrics may be employed for further analysis and generic scoring of the behaviour patterns.

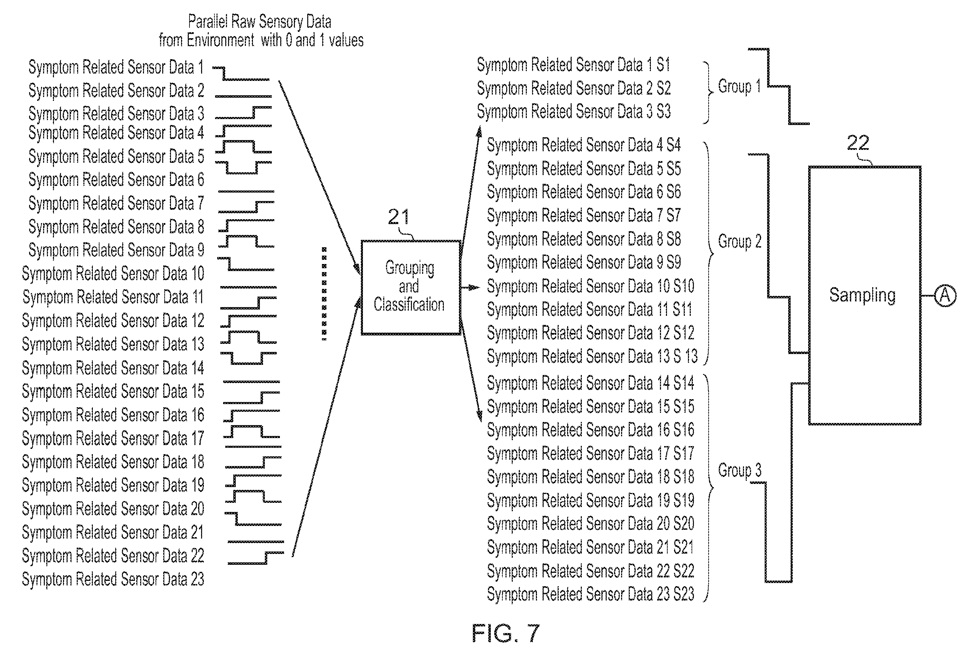

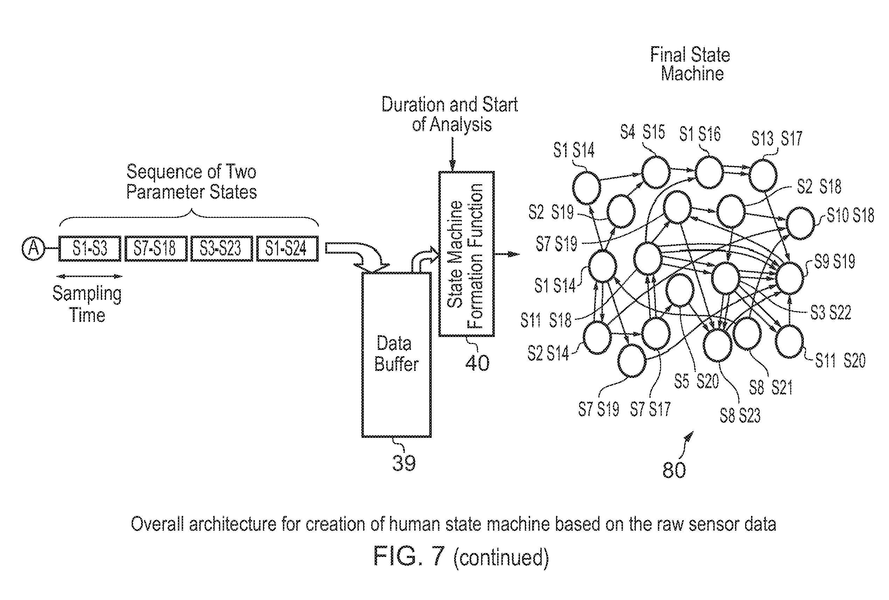

The overall architecture of a preferred embodiment is depicted in Figure 7.

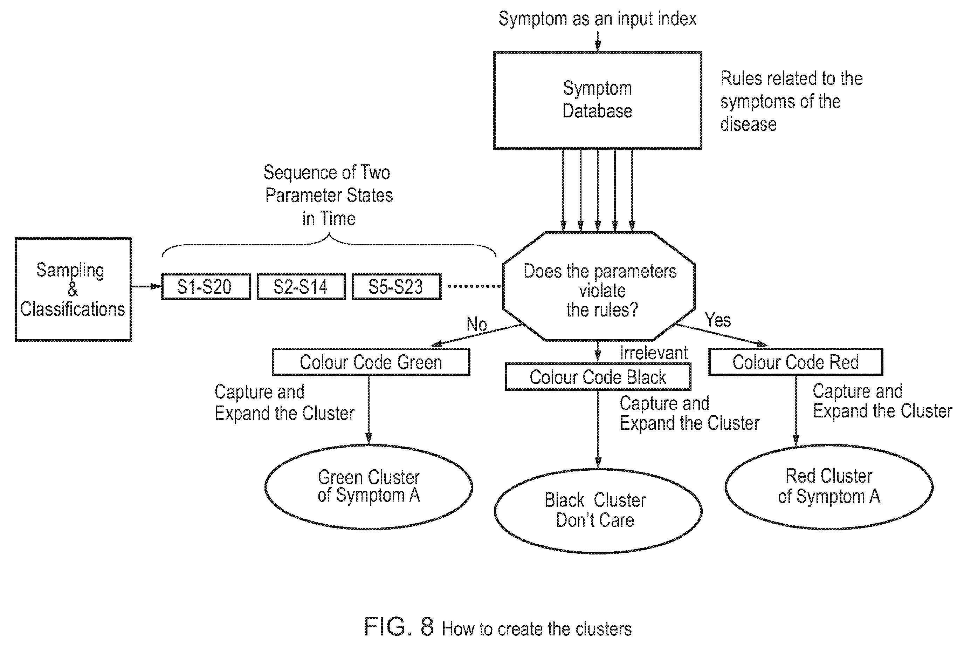

Invention embodiments may establish disease related clusters and an overall cluster space for a human state machine. Figure 8 is a schematic diagram showing clustering of the sequence of two parameter states in time. The two parameters states are fed to a decision unit which decides whether the parameters violate the rules, whether they comply with the rules or whether they are irrelevant to the rules. The rules are fed from the symptom database which is filled with symptoms. Any state which is in compliance with the rules is coloured green or given some other marking. If there is no specific contradiction or agreement between the rules and the state, the state is marked with black or another differentiated marking. Finally, if the parameters in the state violate the rules then the state is colour coded red or otherwise marked to differentiate from the other categories.

In order to establish symptom related clusters, the functions shown in the block diagram of Figure 8 may be used.

This functioning processes the human state machine to establish identifiable clusters in the human state machine for example using colour coding when it is displayed. A database of rules will be employed. For different diseases, medical experts can define different databases of rules. They can also define what states should be classified as Don't Care or which ones violate the rules.

**Monitoring of Alzheimer's Disease and Memory Loss**

This section provides detailed examples and explanation as to how the basic architecture and functionality of invention embodiments can be applied for the detection of memory loss and Alzheimer's. As an example we apply the Human State Machine to the detection of the Alzheimer's disease.

Within the group of the elderly, memory loss and Alzheimer's is a common disease. This group of patients is vulnerable to all sort of potential problems arising from the memory loss. Invention embodiments aim to guarantee that the symptoms of Alzheimer's disease and memory loss are detected well in advance, allowing the elderly to live alone and receive the medical attention they need, while maintaining their independence and freedom. One aim is to find the daily activity pattern or model of a person, for example using a wireless sensor network and then to discover the deviations from normal behaviour patterns. For example passive sensor networks including infrared sensors (PIS) can be applied.

Another possible alternative or additional aim is also to make sure that the person carries out the specific exercises assigned by their doctor (General Practitioner (GP in the UK)) or care staff, engaging (or not engaging) in the activities that the doctor recommends (or does not recommend) for example taking medicines or vitamins on time, preventing the further progress of abnormalities in the behaviour, discovering group behaviour patterns and the person's behaviour pattern within the group and whether or not interaction with the group has a positive impact on person's behaviour pattern progress.

Also it may be possible to detect unusual or abnormal activity and notify the necessary medical personnel and/or family and close friends with detailed information about the status of the monitored person, over the most recent few minutes, hours or days, and more. Another possibility is that the immediate family members and/or medical personnel might be able to configure and re-task the network based on the discovered abnormal behaviour and advice received, in order to adjust the monitoring process and customize it to the needs of the elder person over time.

**Part I: The Basic Architecture**

The following section describes the syndromes of Alzheimer's disease, from results of studies at the Johns Hopkins Medical Centre.

- Increasing forgetfulness and short-term memory loss.
- Difficulty making decisions.
- Impaired judgment; new difficulty making mathematical calculations or handling money.
- Decreased knowledge of current events.
- Anxiety, withdrawal, and depression as awareness of deficits becomes frightening and embarrassing.

- Language difficulties, including rambling speech, frequent inability to name familiar objects, long pauses to find the right word, and repetition of the same words, phrases, or questions.
- Loss of ability to communicate verbally or to write and understand written language.
- Delusions, hallucinations, paranoia, or irrational accusations.
- Agitation and combativeness.
- Unusual quiet and social withdrawal.
- Wandering or getting lost in familiar places.
- Urinary and fecal incontinence.
- Inappropriate social behaviour; indifference to others.
- Failure to recognize friends and family.
- Inability to dress, eat, bathe, or use a bathroom without assistance.
- Walking difficulty or multiple falls.

**Selecting Sensory Data Linked to Memory Loss and Alzheimer's Disease Symptoms**

The diagnosis of Alzheimer's disease is based on patient history (including input from family members) and clinical examination, including a test of mental status. It is recommended that a doctor be called when symptoms of memory loss start to show. In this embodiment we aim at automating some of this process by introducing simple tests that can be undertaken with the sensors to check the status of memory loss of patients. We will start by mapping Alzheimer's symptoms (listed by Johns Hopkins Medical Centre, as above) into simple sensory information, preferably gathered in a seamless and non-intrusive way. These parameters are grouped into three sensory information categories to reflect the Johns Hopkins Medical Centre findings on Alzheimer's disease symptoms:

**Group One: Walking/Gait Status "How is your walking?"**

1. **Normal Walking, NW**
2. **Walking with Difficulty, DW**
3. **Fall, FW**

Group one is to cover and detect the last symptom in the Johns Hopkins list, i.e. walking difficulty or multiple falls. Walking and fall status can be measured both in a physically intrusive (i.e. using wearable sensors) or non-intrusive (i.e. using video surveillance technologies) ways.

**Group Two: Whereabouts of the Patient at each point of time**

The rooms: A 3 Bedroom Apartment with

1. **Living Room, LV**
2. **Office Room, OR**
3. **Bath Room, BT**
4. **WC, WC**
5. **Kitchen, KI**
6. **Bedroom, BR**
7. **Balcony, BA**
8. **Lounge, LO**
9. **Outside Home, OH**

Group two is to cover and detect multiple symptoms in the Johns Hopkins list, i.e.:

- Increasing forgetfulness and short-term memory loss.
- Difficulty making decisions.
- Impaired judgment; new difficulty making mathematical calculations or handling money.
- Decreased knowledge of current events.
- Wandering or getting lost in familiar places.
- Inability to dress, eat, bathe, or use a bathroom without assistance.

**Group Three: The Activities of the Patient**

Using a floor plan including the rooms listed above, activities may include:....

**1. Exercise A in Living Room (EA): For example standing at spot 'A' for 10 minutes at a Specified time**
**2. Exercise B in Office Room (EB): For example standing at spot 'B' for 10 minutes at a specified time**
**3. Exercise C in Kitchen (EC): For example standing at spot C for 10 ,minutes at a specified time**
**4. Exercise M (EM) Taking Medicine at a Specified Time**
**5. Exercise V (EV): Taking vitamins at specified time**
**6. Exercise F in Internet Surfing (IS)**
**7. Exercise G in Watching TV (TV)**
**8. Exercise H in Communication (CO)**
**9. Sleep (SL)**
**10. Any Other Non Specified Activity (NS)**

Group three is to cover and detect multiple symptoms in the Johns Hopkins list, i.e.:

• Increasing forgetfulness and short-term memory loss.
• Difficulty making decisions.
• Impaired judgment; new difficulty making mathematical calculations or handling money.
• Decreased knowledge of current events.
• Wandering or getting lost in familiar places.
• Inability to dress, eat, bathe, or use a bathroom without assistance.
• Loss of ability to communicate
• Unusual quiet and social withdrawal.

This example creates a human health signature (HHS) based on a human state machine as a result of the sensor data gathered and usable for both short-term and long-term behaviour monitoring for each human subject.

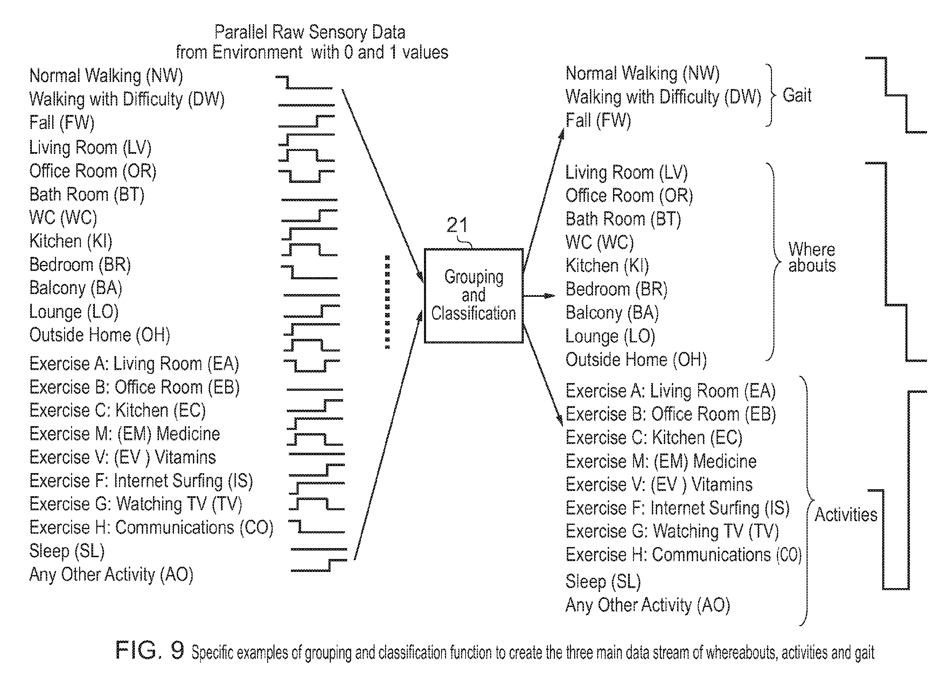

The grouping and classification function detailed in the general embodiment is used to group the raw sensory data and with values 0 and 1, into three main streams of data for whereabouts, activities and gait as shown in Figure 9. Basic explanation of this diagram is omitted, and the reader is referred to the description of Figure 3, which is a general figure for relevant explanation.

The grouping and classification function is a simple mechanism that identifies the nature of the incoming binary data and classifies it into three groups: Gait, Whereabouts and Activities. In doing that it directs the incoming binary data into three main data streams of whereabouts, activities and gait.

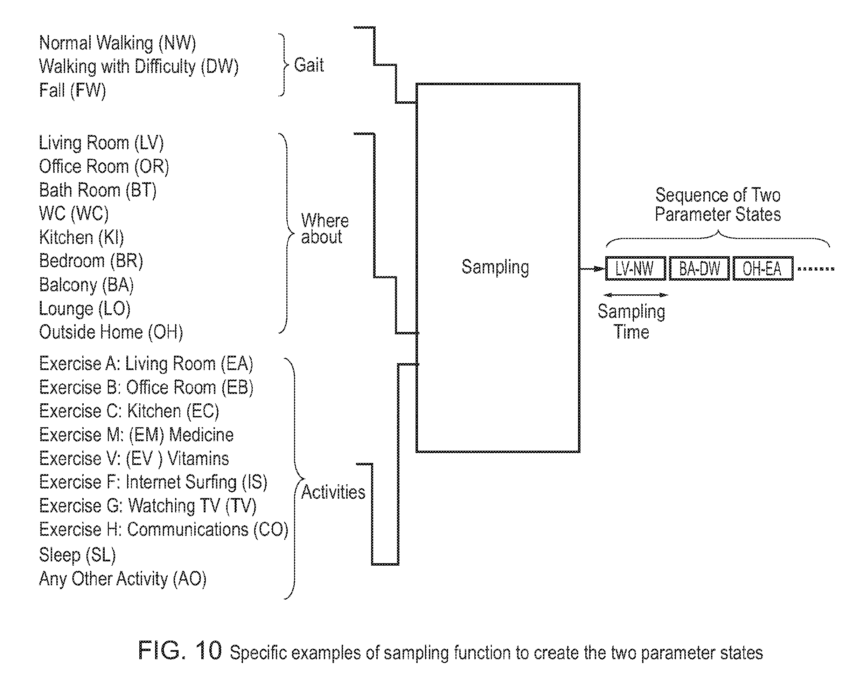

The specific sampling function is shown in Figure 10, creating a two-parameter state by sampling the three data streams related to whereabouts, activities and gait, which are the outputs of the grouping function at certain points of time. The reader is referred to the description Figure 4 for a generic explanation of the Figure 10 concepts.

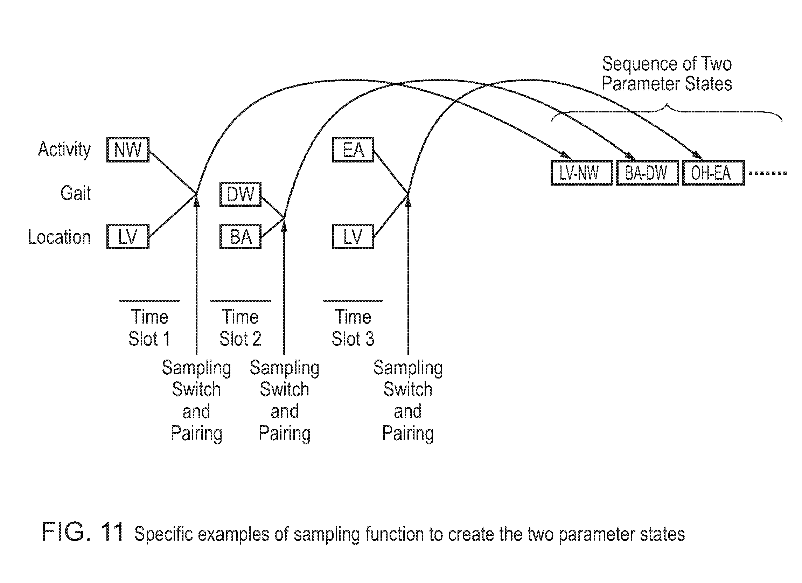

The specific sampling function is illustrated in Figure 11. At each point of time the sampling function takes a sample of whereabouts and either one of gait or activity and forms the two-parameter states. The reader is referred to the description of Figure 5 for a generic explanation. The two parameter state OH-EA (outside home, exercise in living room) indicates that exercise is being performed, but not in the living room, it is a problematic behaviour.

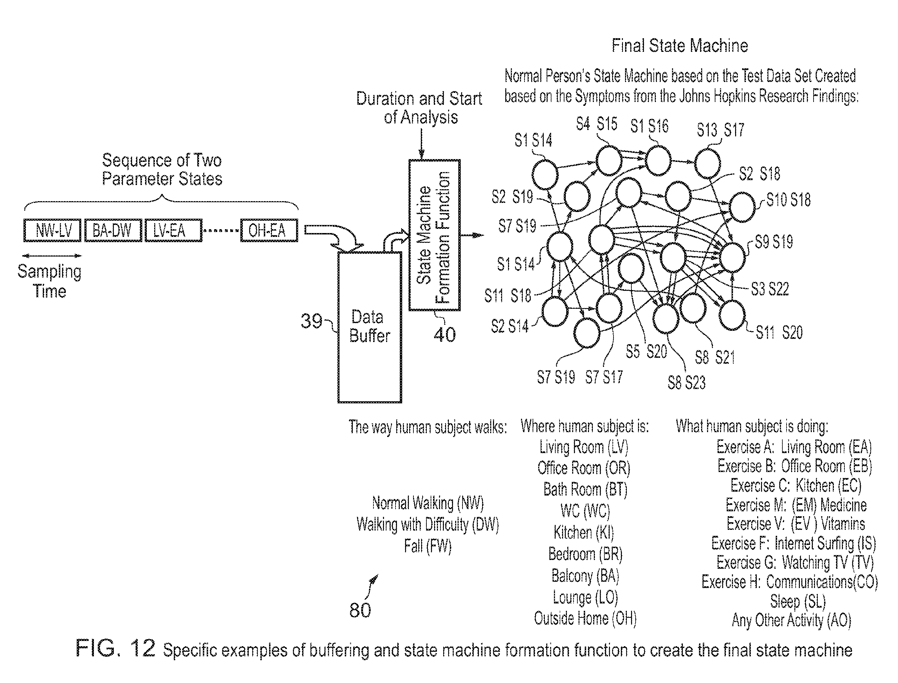

Finally, the extracted sequence of the states is buffered. Based on the length of time and the starting point of the analysis, as input parameters, the state machine formation function scans through the buffered data and creates a state machine and determines its related probabilities as shown in Figure 12. (The reader is referred to the corresponding generic figure description of Figure 6.)

For the current state machine and for each state we calculate the ratio of the time spent to the total period and the total probability of transitions to other potential states as before.

**MATLAB Implementation of the Architecture and Simulation Results**

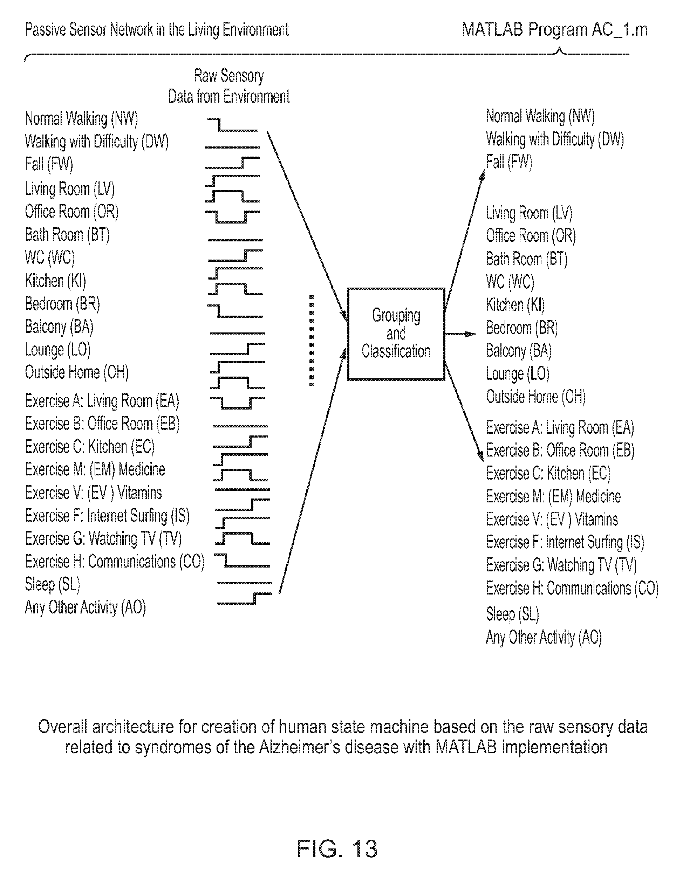

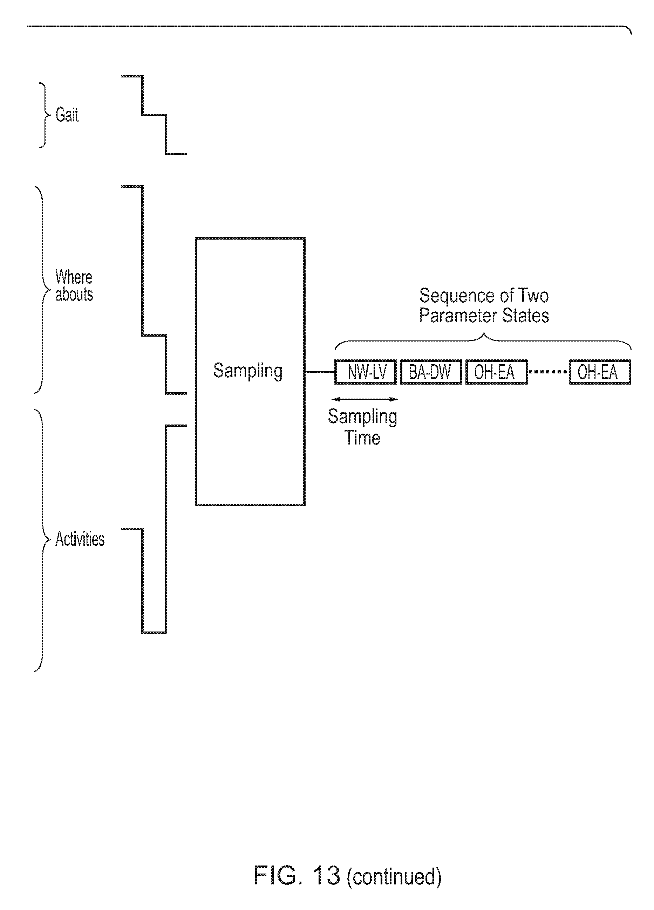

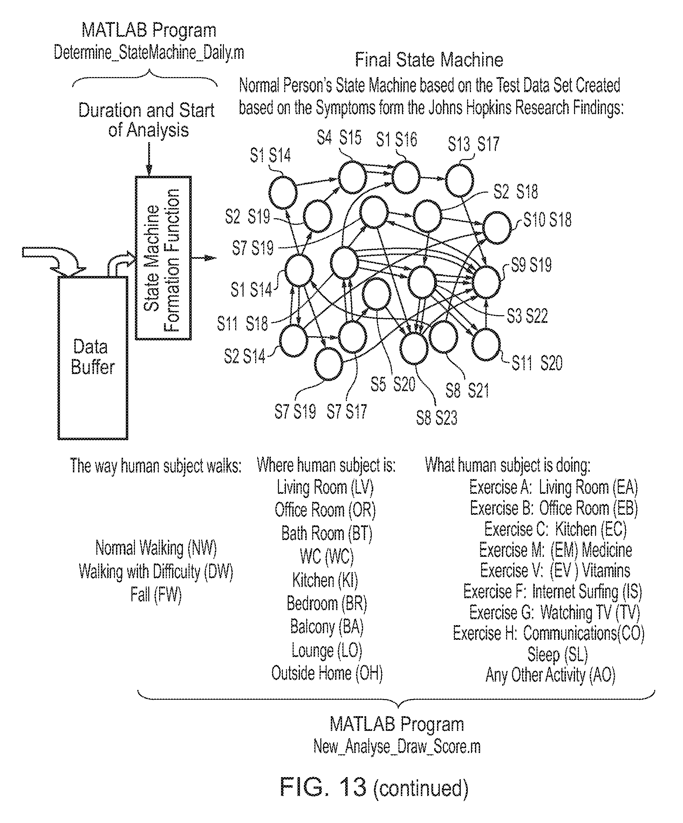

A simulation platform was created in MATLAB (a numerical computing environment) to realise the architecture explained above. Different software modules were linked to the different parts of the architecture as shown in Figure 13.

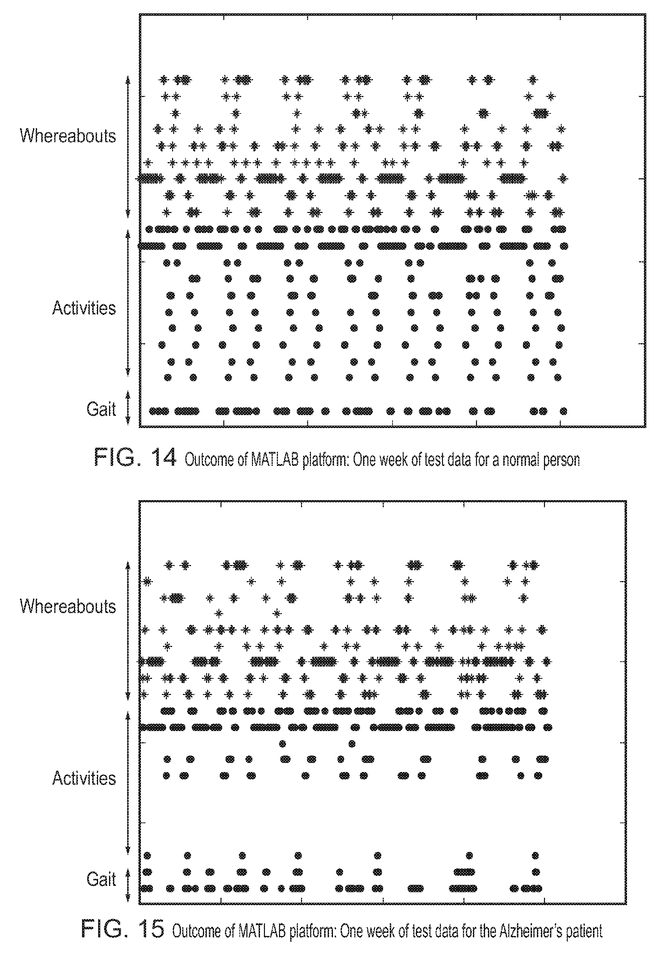

In order to test the architecture, digitization of test data based on the scenarios for a normal person and an Alzheimer's patient respectively, using the Johns Hopkins' findings (i.e. the identified syndromes) for 7 days were completed in MATLAB. The final data after grouping, classification and sampling is shown below for a normal person (Figure 14) and an Alzheimer's patient (Figure 15). These graphs are based on a two-dimensional grid of whereabouts, activities or gait mapped against time.

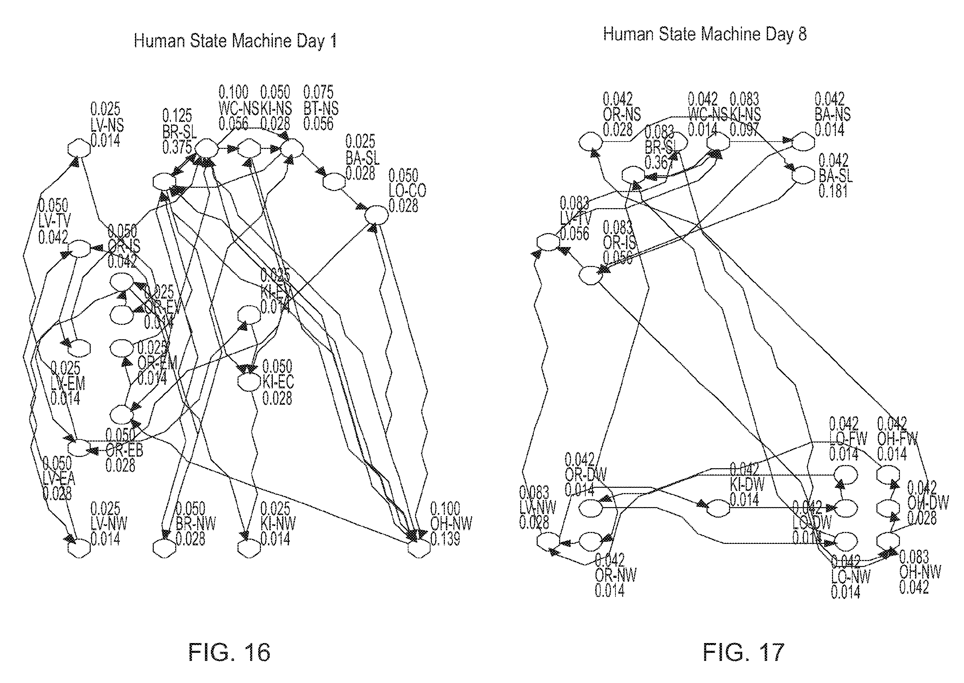

The MATLAB platform is capable of creating the human state machine for a daily or a weekly or any other time scale basis. The MATLAB platform is capable of showing the changes to the state machines on the fly as the days pass by including changes to a state machine on a per day basis, including the changes to the probabilities of state transitions, all new states and state transitions, including outgoing states. The outcomes of an analysis using the MATLAB platform analysis for 1 day for a normal person and an Alzheimer's patient based on the test data are shown in figures 16 and 17 respectively.

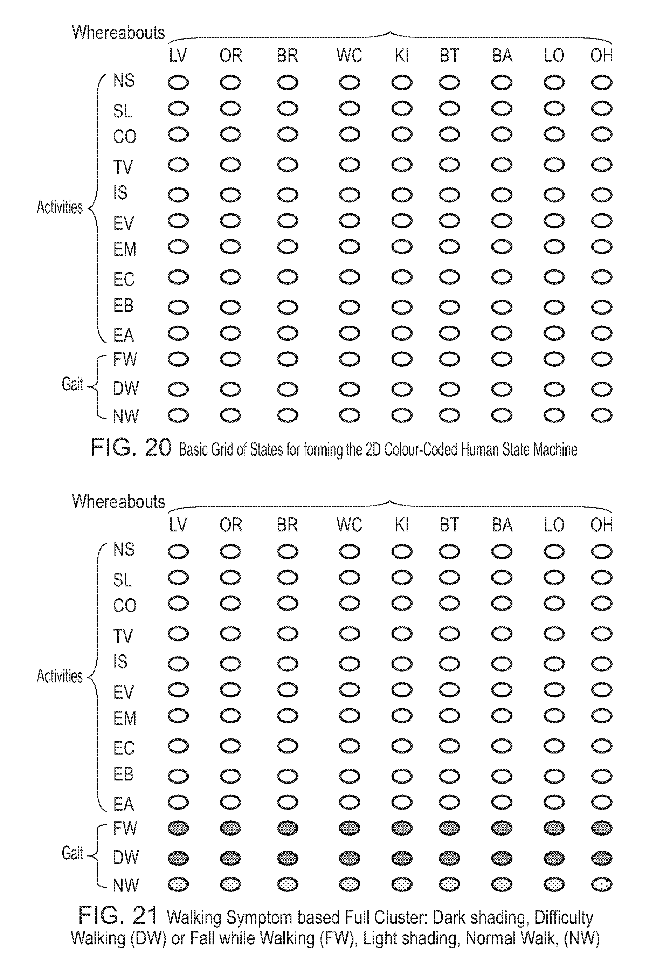

These two figures are a representation of the human state machine. Each point shown is a two-parameter state. For example the bottom left hand point LV - NW represents the state in which the subject in the living room with normal walking. The figures shown above and below LV - NW are the total probability of transitions to other potential states and the ratio of time spent to the total period respectively. The position of the point in the diagram is based on a 2D grid in which the whereabouts from the x axis and the gait and activities from the Y axis of the grid. The grid and its potential activities is shown for example in figure 20.

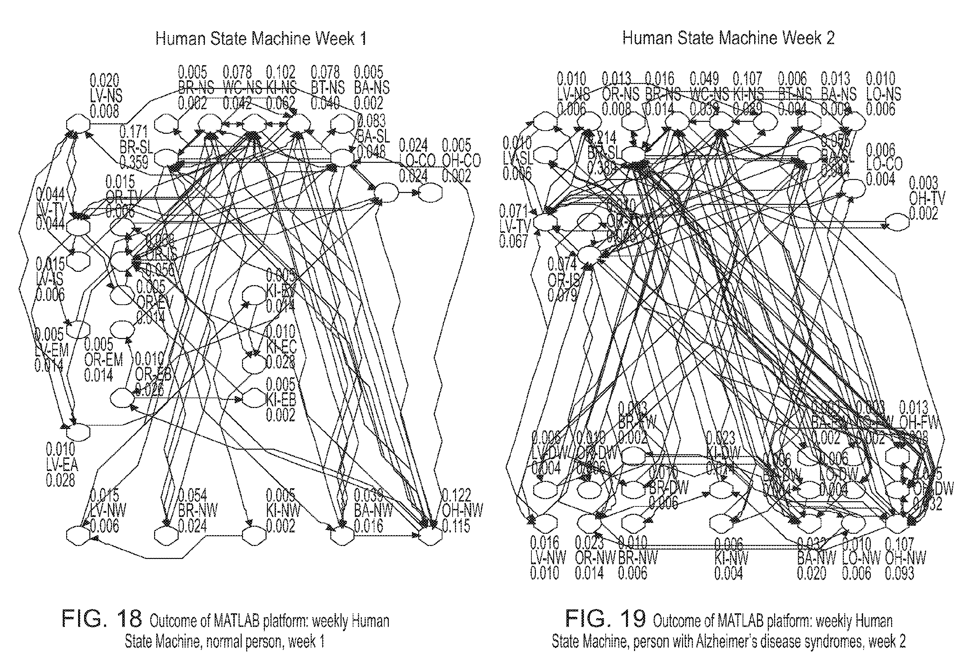

The outcomes of the MATLAB platform analysis for a weekly human state machine for two different weeks are shown in Figures 18 (normal) and 19 (Alzheimer's).

**Part II: Color Coded Human State Machine**

This section describes a 2D color coded human state machine for prediction and diagnosis of memory loss and Alzheimer's disease, but the same techniques can be used for other examples

In establishing a colour code human state machine (HSM) and the related clusters, the following steps maybe taken:

1. We assume that each state is an object which contains multiple parameters such that:

$$\mathbf{S} = \{S_k\} = \{Loc_m, Act_n, \{Sin_s\}, \{Sout_d\}, \{Tstart_i\}, \{Tspent_i\}\}, m = 1\cdots M, n = 1\cdots N, \tag{3}$$
$$s = 1\cdots S, d = 1\cdots D, i = 1\cdots I, k = 1\cdots m\cdot n$$

where $\{S_k\}$ represents the overall space of sates, $S_k$ represents each state within the total space of states, $Loc_m$ represent the location of the human subject at each point in time, $Act_n$ is the activity undertaken, $\{Sin_s\}$ represents the set of states with transitions into the state $S_k$, and $\{Sout_d\}$ represents the destination states that the state $S_k$ has made transitions to, $Tstart_i$ is the start time of each activity, $Tspent_i$ is the total time spent on i different occasions, $M$ is the total number of locations, $N$ is the total number of activities, $S$ is the total number of states with transitions into the state $S_k$, $D$ is the number of destination states that the state $S_k$ has made transitions into them, $I$ is the total number of occurrences per states and k is determined as $k=(m-1).N+n$.

2. A state machine on its own may only show the status of whereabouts and activities and the transition between the states and the related probabilities. We define a space of symptoms of the disease to be linked to the state space (which includes all the potential states which might be detected), in order to perform detection or diagnosis of the disease. The space of symptoms is defined as:

$$\mathbf{Sympt} = \{Sympt_l\}, \qquad l = 1\cdots L \tag{4}$$

3. A cluster is a set of visible states or their relevant transitions which has been linked to the specific symptoms following some predefined rules:

$$\mathbf{C} = \{\{S_t\}, \{S_q\}, \{R_h\}\}, \qquad t = 1\cdots T, q = 1\cdots Q, h = 1\cdots H, \mathbf{C} \subset \mathbf{S} \tag{5}$$

where $\{S_t\}$ are the set of states forming the potential hot spot problem area states, T indicates the number of states within $\{S_t\}$, $\{S_q\}$ are the set of states or their transitions which form the must-have areas (i.e. set of states which indicate the person does not have a disease), Q indicates the number of states within $\{S_q\}$, and $\{R_h\}$ are the set of rules which relates the states to the specific symptoms and $H$ is the total number of rules. The states carried within a cluster are a subset of a HSM.

4. We consider a 2D plane to draw a human state machine. The 2D space is divided into a grid. The vertical axis shows the activities and the horizontal axis shows the whereabouts, as shown in Figure 20. This is in effect an overview or system model representation, showing all possible combinations of whereabouts with activities and gait, for example the set of must-have states and trouble hot spot areas are shown in Figure 21 for the walking status.

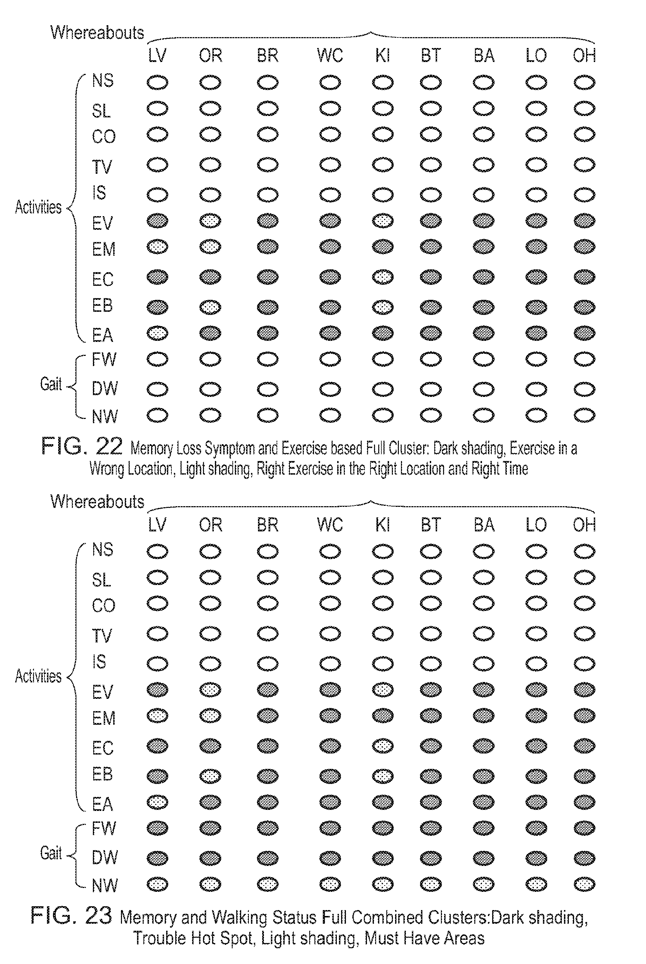

The set of must-have states and trouble hot spot areas are shown in Figure 22 for the memory loss and the related exercises:

A combined version of the memory and walking status cluster is shown in Figure 23.

**A 3D representation of the Color Coded Human State Machine for Prediction and Diagnosis of the Memory Loss and Alzheimer's Disease**

A 3D representation of the color coded Human State Machine for prediction and diagnosis of the memory loss and Alzheimer's disease can be a more accurate way of showing the trouble hot spots when critical timing of the activities matters in addition to the location. The same representation can be used for other examples of diseases/medical conditions with different parameters matching the selected symptoms as necessary.

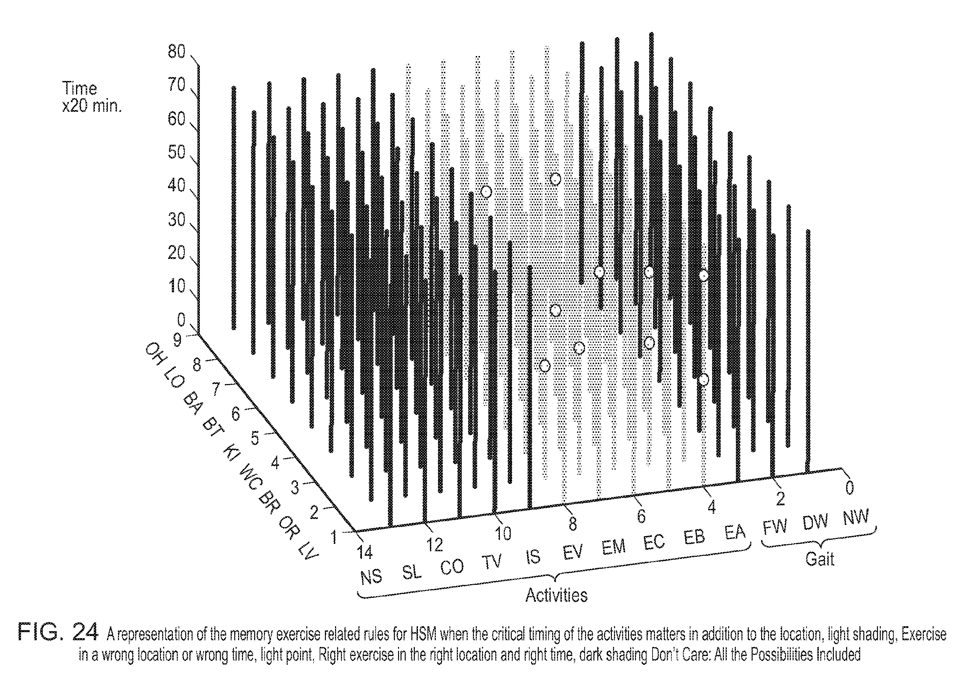

For example in memory loss, when the exercise only in specific point of time and specific location is recommended, a better visualization of the clusters is a 3D representation of the state machine shown in Figure 23 where exercise in a wrong time, in a wrong location or both is visible. Here, lighter shading identifies exercising correct behaviour (which might be green on a colour display) and darker shading identifies incorrect behaviour (which might be red on a colour display). It is assumed that the vertical axis is time representing n=72 cells of $\Delta t$=20 minutes during 24 hours as shown in Figure 24. Figure 24 shows all the possibilities within an overview/system model.

**How to Establish the Clusters and the Overall Cluster Space for a Human State Machine: The Functional Block Diagram**

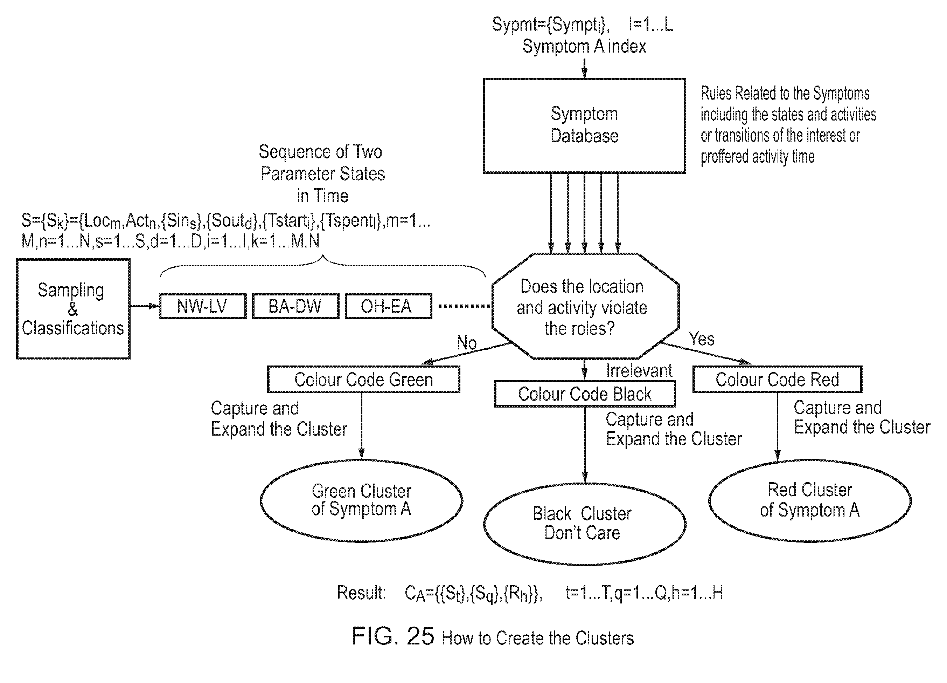

In order to establish a cluster we propose the functional block diagram in Figure 25. Figure 25 is a specific diagram corresponding to the general diagram of Figure 8.

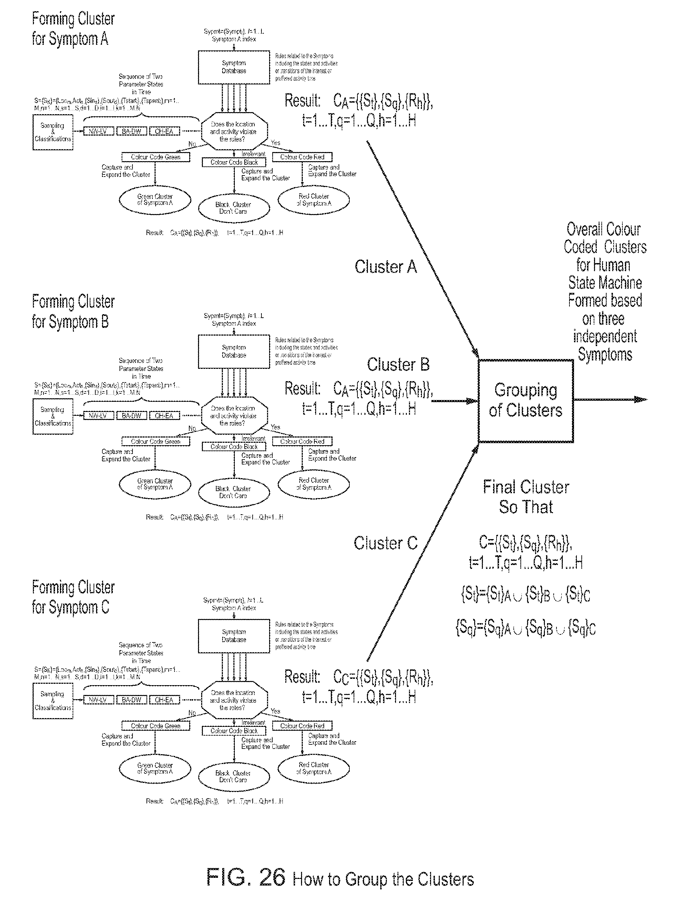

The clusters from all the symptoms may then be combined to form the overall trouble hot spots and the trouble free region as shown in Figure 26. Figure 26 is the combination of cluster forming for 3 distinct symptoms, symptoms A, B and C leading to a final cluster.

**MATLAB Implementation of the Colour Code State Machine and Simulation Results**

A simulation platform was created in MATLAB to realise the architecture of some invention embodiments.

**A Test Scenario for Progress of the Alzheimer's Disease Symptoms**

Based on a random diversification of the progress of the Alzheimer's disease symptoms the following table is created for 30 weeks. 5 weeks only of results are shown below. The following scenarios were considered:
1. EA None
2. EB None
3. EC None
4. EM None
5. EV None
6. No CO
7. DW FW
8. No BT
9. No OH
10. Wander in Night
11. Missed Regular TV
12. Missed IS

| | Day 1 | Day 2 | Day 3 | Day 4 | Day 5 | Day 6 | Day 7 |
|---|---|---|---|---|---|---|---|
| Week 1 | 1 | 2 | 3 | 4 EV Wrong Time | 5 | 6 | 7 |

(continued)

|  | Day 1 | Day 2 | Day 3 | Day 4 | Day 5 | Day 6 | Day 7 |
|---|---|---|---|---|---|---|---|
| Week 2 | 8 EA Wrong Time | 9 EB Wrong Time | 10 EM Wrong Time | 11 | 12 | 13 EV Wrong Time | 14 |
| Week 3 | 15 EM Wrong Time | 16 | 17 EC Wrong Time | 18 | 19 EA Wrong Time | 20 EB Wrong Time | 21 EV Wrong Time |
| Week 4 | 22 EB Wrong Time | 23 EC Wrong Time | 24 EM Wrong Time | 25 EV Wrong Time | 26 EB Wrong Time | 27 | 28 EA Wrong Time |
| Week 5 | 29 EC Wrong Time EA Wrong Time | 30 EA Wrong Time EB Wrong Time | 31 EB Wrong Time EM Wrong Time | 32 EA Wrong Location No Co No BT | 33 EV Wrong Time | 34 EC Wrong Time EV Wrong Time | 35 EM Wrong Time |

The scenarios are then reverse coded (that is, suitably translated) into the MATLAB platform so that when the program runs, a dynamic growth of the symptom-related problems can be observed. It is assumed that the algorithms are blind in a sense that the only input is the binary sensory data and the database of the Alzheimer's symptoms.

The colours used in the circles of the 2D state representation on a colour display:

  **1. Black (shown as points in the figures):** Don't Care
  **2. Red (shown as asterisks in the figures):** Problematic states of hot spot problem areas
  **3. Green (shown as t crosses in the figures):** Exercise carried out successfully, or positive behaviour patterns

The colours in 3D time-location-activity representation on a colour display and corresponding representation used in the figures:

  **1. Blue (shown as points and surrounding circle):** overlap between the two behaviour clusters: Missed Exercise and problematic walking (DW and FW)
  **2. Black (shown as points):** Don't Care actions
  **3. Red (shown as asterisks):** Exercise in Wrong Time or Wrong Location
  **4. Magenta (shown as x crosses):** Difficulty with walking (FW or NW)
  **5. Green (shown as t crosses):** Exercise carried out successfully

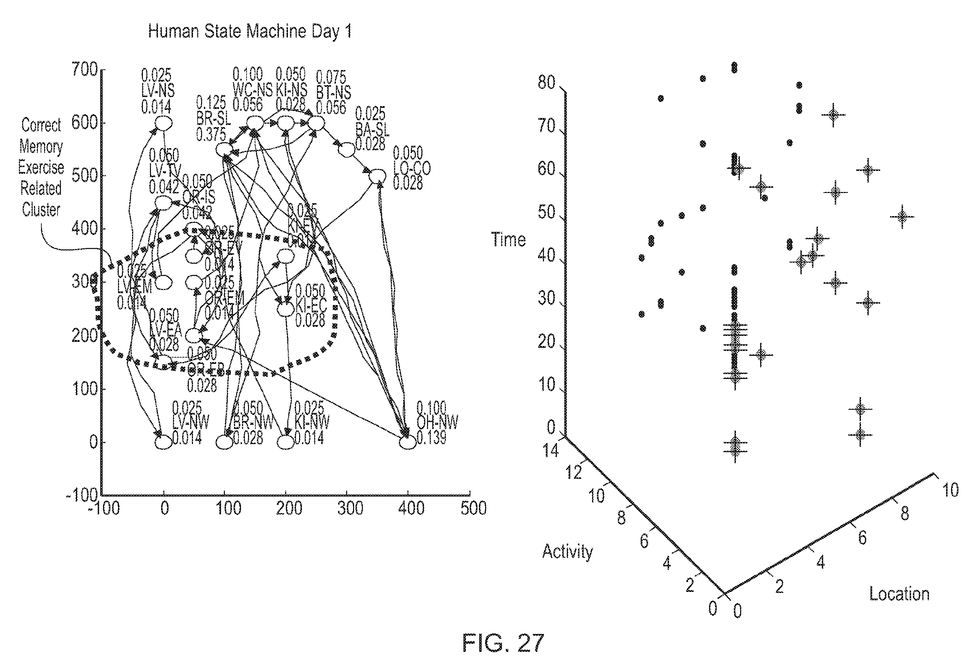

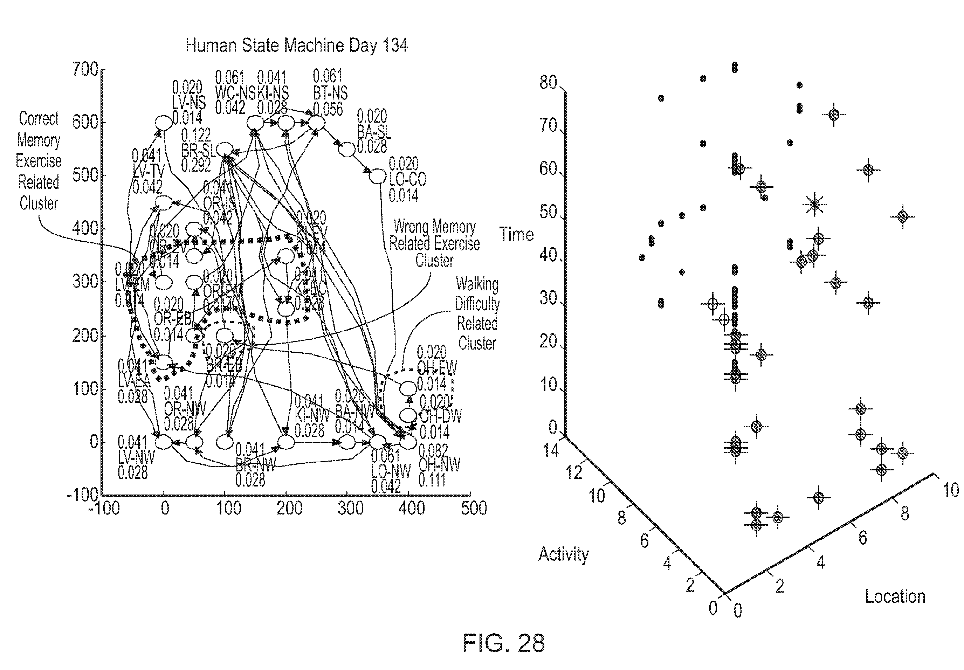

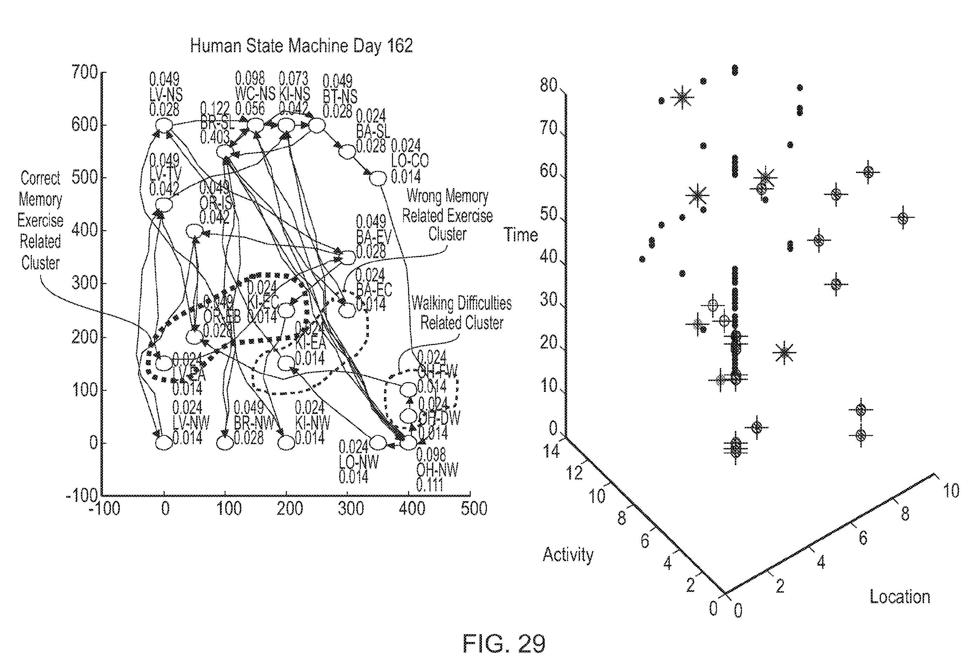

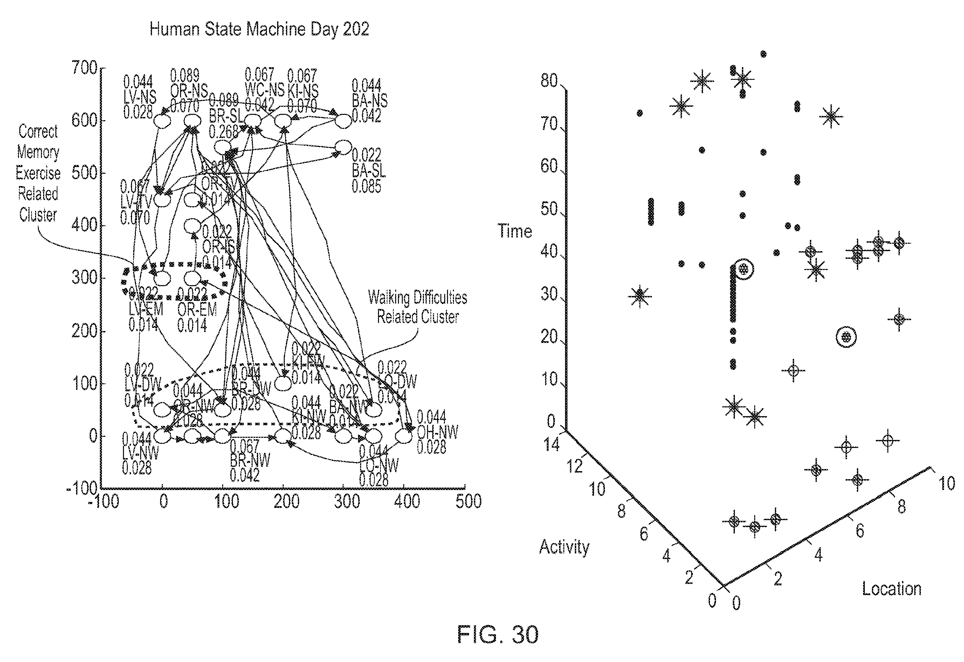

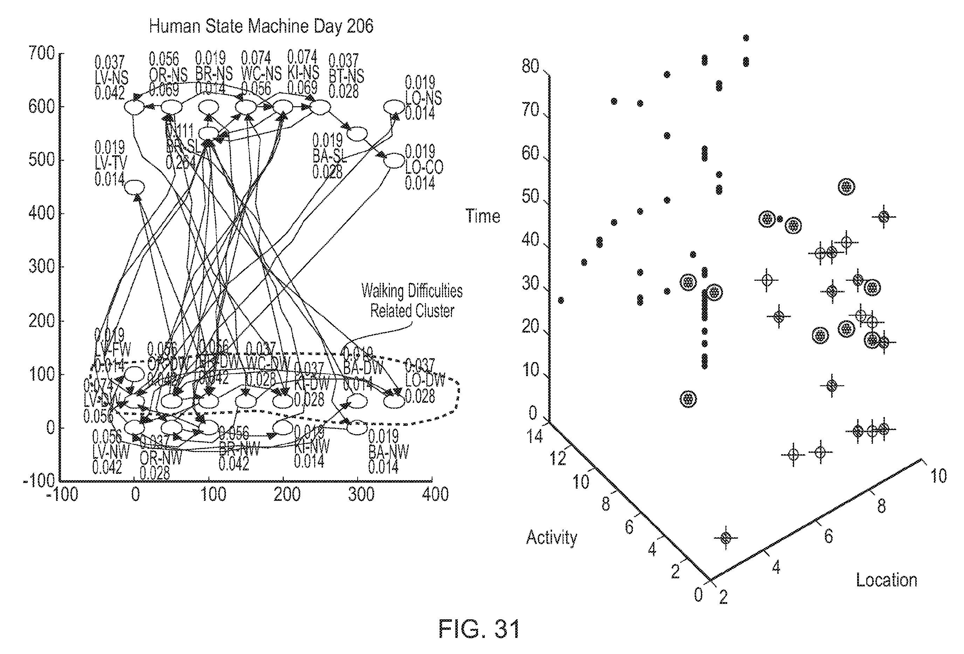

The following simulation results, presented in Figures 27 to 31, shows the progress of symptoms and their related colour coded state machine on a daily basis. Namely it can be seen how the cluster related to the memory exercises in the right location and the right time (which would be a green cluster) is shrinking and the cluster related to the walking difficulties (which would be a red cluster) is growing on a daily basis. It can be seen that how we have detected and visualised the progress of the Alzheimer's disease. The 3D Time-Location-Activity illustration provides an insight to the progress of the symptoms in time.

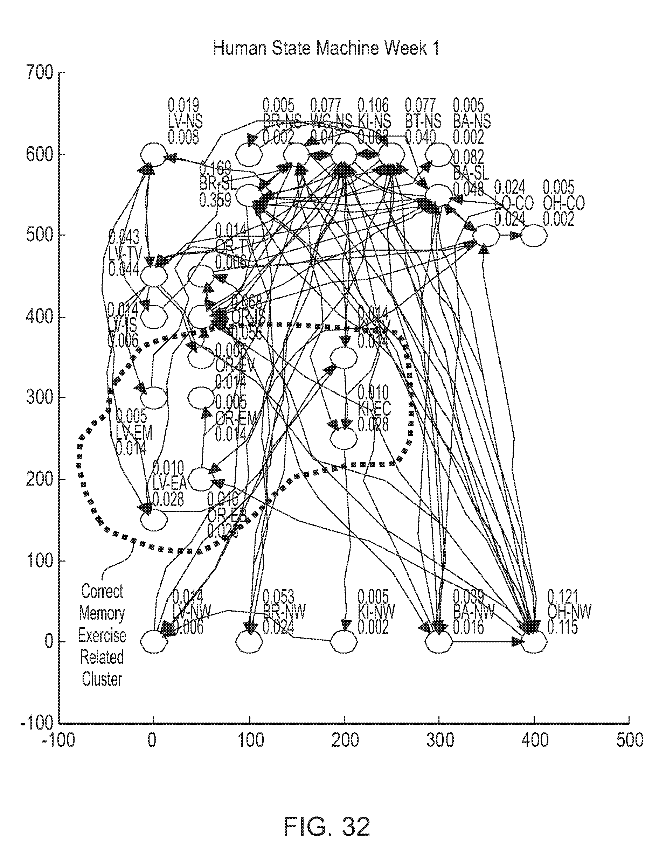

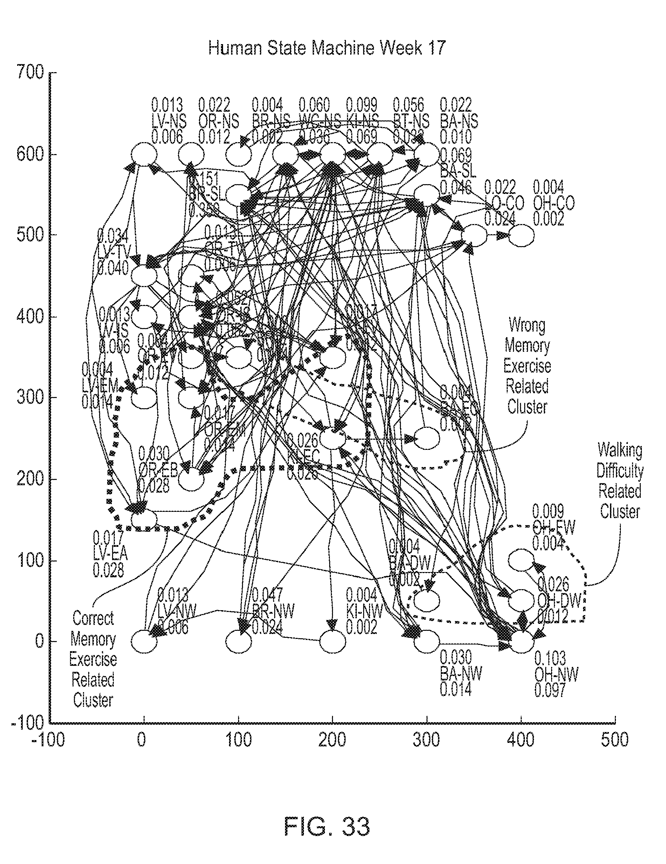

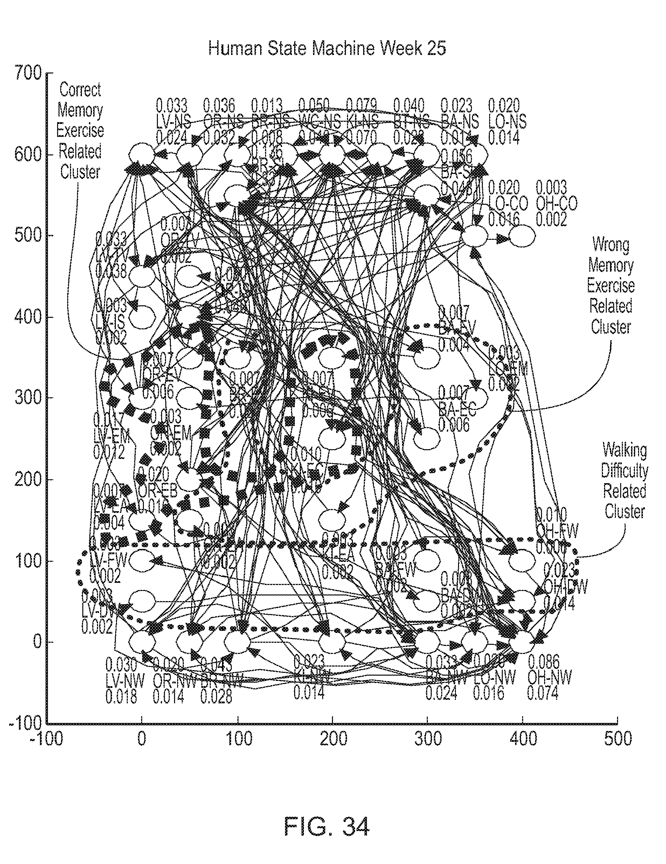

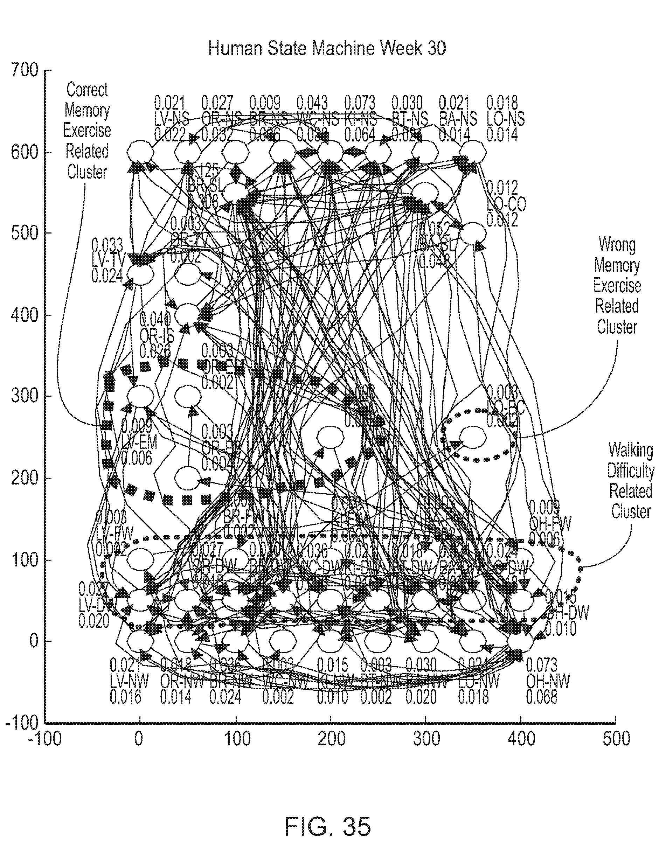

In Figures 32 to 35 the progress of the symptoms is shown on a weekly basis. With "red" and "green" clusters highlighted and labelled.

In summary, invention embodiments provide an architecture and function flow to take disease symptom-related raw sensor data, and convert it into an N-dimensional flexible grid of a human state machine.

Some embodiments describe a process-chain to establish a colour coded clustered human state machine out of that human state machine based on the disease-symptoms-related rule database by establishing colour-coded clusters.

Some invention embodiments can tie the states of the human state machine to compulsory symptom-related exercises, in an innovative fashion. The concept of the flexible N-dimensional grid which can be tailored to cover N different parameters related to any disease symptoms is an innovative feature of many different embodiments. The elements within the different dimensions of grid of the human state machine may be defined (for example each specific row or column may have a symptom related meaning) based on the disease symptoms. The human state machine undergoes dynamic changes over time based on sensor data changes.

Based on simple sensory data, embodiments provide a human state machine focussed on the syndromes of a disease and a powerful visualization tool for monitoring the progress and early detection of the disease. The invention embodiments provide the potential for an automated, seamless and non-intrusive detection of different diseases.

Employing a simple set of input binary sensory data, a colour-coded (or otherwise differentiated) human state machine may be created, based on the clusters of human behaviour related to the symptoms of the disease.

The proposed colour state machine and the clusters make it possible to monitor the progress of the problematic behavioural pattern or hot spot behaviour problem areas, indentifying daily or monthly shift of behavioural clusters and the rate the clusters may grow in time. These all can lead to a more accurate seamless care in which the colour-coded data identifies the problematic areas and can be used by the medical experts to diagnose the progress of the disease in a very simple and powerful visualised way.

In a more sophisticated application of the colour-coded human state machine, it is also possible to monitor the causes, events or the reasons and specifically the transitions from different clusters of behaviours into each other to identify what has led to the growth of problematic behaviour clusters. It may be employed to study the historical trend of the growth of the problematic behaviour clusters and the surrounding historic circumstances which has led to the growth of the problematic behaviour clusters i.e. the worsening situation with the disease sufferer.

**Claims**

1. A method for analysing sensor data in the form of parameters describing the condition of a subject, the method comprising:

    processing the sensor data to provide, at each of a plurality of time instants, a collection of two or more of the parameters, each of which parameters describes a different aspect of the subject's condition, the parameters together representing a state of the subject;
    putting together successive collections to form a sequence of states; and
    analysing the sequence to create a state machine representing the states of the subject detected over a period of time comprising the plurality of time instants.

2. A method according to claim 1, wherein the processing comprises:

    grouping the sensor data into groups, each group including all the parameters describing a single aspect of the subject's condition and the parameters in each group being mutually exclusive so that the group provides a data stream of the parameters successively present over time, and
    sampling the data from at least two data streams at different time instants, to form a state represented by a collection of the parameters which are present at each time instant, each collection including the parameter from each data stream that is sampled at that time instant.

3. A method according to claim 1, further comprising
    selecting sensor data which is relevant to one or more diseases or medical conditions to be investigated before inputting the sensor data; and/or
    inputting and pre-processing the sensor data, the pre-processing binarising any non-binary values in the sensor data and providing mutually exclusive binarised parameters for the processing.

4. A method according to any of the preceding claims, wherein
    the aspects included within the collections vary to give two or more different collection types, the sequence of collections preferably alternating between two different collection types, and preferably wherein
    the collection type changes at each new time instant, or at every second time instant.

5. A method according to any of the preceding claims, wherein
    analysing the state machine includes storing all the states detected over the period of time and the transitions between them, and preferably includes calculation of the total time spent in each state and the probability of transition to other detected states.

6. A method according to any of the preceding claims, wherein
    the state machine is displayed based on a grid of potential states, with N axes, an axis for each of N aspects displayed, the states detected being represented on the grid, transitions between states preferably also being represented; and/or wherein

the state machine is displayed based on a grid of potential states and time, with N+1 axes, an axis for each of N aspects displayed and an axis for time, the states detected and their timing being represented.

7. A method according to any of the preceding claims, wherein
   each state detected in the state machine or each potential state of a system model is compared to a database of medical rules, and preferably wherein a warning is provided if the state does not conform to the rules, and/or the database of medical rules is related to symptoms of one or more diseases or medical conditions; and/or each rule corresponds to at least one symptom of the disease or medical condition.

8. A method according to claim 7, wherein
   the result of the comparison is a classification of each state as in violation of, in line with, or not relevant to one or more of the rules, and wherein each state is marked accordingly.

9. A method according to claim 8, wherein
   the marking is provided on a representation of the states, with one marking for any state in violation of on or more of the rules, another marking for any state in line with one or more of the rules and a third marking for any state not relevant to one or more of the rules.

10. A method according to any of claims 7 to 9, wherein
    the states in a system model are divided into a set of one or more clusters, including any of: a problem cluster for all the states which are in violation of one or more of the rules; a must-have cluster for all the states which are in line with one or more of the rules; and a don't care cluster for all the states which are not relevant to one or more of the rules; and each detected state is automatically allocated to a cluster of the system model; or wherein each detected state is compared with the database of medical rules and the detected states are divided into a set of one or more clusters, including any of: a problem cluster for all the states which are in violation of one or more of the rules; a must-have cluster for all the states which are in line with one or more of the rules; and a don't care cluster for all the states which are not relevant to one or more of the rules.

11. A method according to claim 10, wherein
    a set of clusters is provided for each rule of a grouping of rules corresponding to the symptoms of a disease or medical condition, and two or more sets of clusters are combined to form a set of overall clusters taking into account two or more symptoms.

12. A method according to claim 10 or 11, wherein the states in each cluster are distinguished by colour coding.

13. A state machine apparatus arranged to analyse sensor data in the form of parameters describing the condition of a subject, the apparatus comprising:

    a processor operable to process the sensor data to provide, at each of a plurality of time instants, a collection of two or more of the parameters, each of which parameters describes a different aspect of the subject's condition, the parameters together representing a state of the subject;
    a sequencer operable to put together successive collections to form a sequence of states; and
    an analyser operable to analyse the sequence to create a state machine representing the states of the subject detected over a period of time comprising the plurality of time instants.

14. A sensing system operable to analyse sensor data in the form of parameters describing the condition of a subject, the system comprising:

    an apparatus according to claim 21; and
    one or more sensors which are operable to gather information which can be used to describe the condition of a subject.

15. A computer program which when executed on a computing apparatus causes it to carry out the method of any of claims 1 to 20, or which when downloaded onto a computing apparatus causes it to become the state machine apparatus of claim 21.

**Amended claims in accordance with Rule 137(2) EPC.**

**1.** A method for analysing sensor data in the form of parameters describing the condition of a subject, the method comprising:

processing the sensor data to provide, at each of a plurality of time instants, a collection of two or more of the parameters, each of which parameters describes a different aspect of the subject's condition, the parameters together representing a state of the subject;
putting together successive collections to form a sequence of states; and
analysing the sequence to create a state machine representing the states of the subject detected over a period of time comprising the plurality of time instants.

**2.** A method according to claim 1, wherein the processing comprises:

grouping the sensor data into groups, each group including all the parameters describing a single aspect of the subject's condition and the parameters in each group being mutually exclusive so that the group provides a data stream of the parameters successively present over time, and
sampling the data from at least two data streams at different time instants, to form a state represented by a collection of the parameters which are present at each time instant, each collection including the parameter from each data stream that is sampled at that time instant.

**3.** A method according to claim 1, further comprising
selecting sensor data which is relevant to one or more diseases or medical conditions to be investigated before inputting the sensor data; and
inputting and pre-processing the sensor data, the pre-processing binarising any non-binary values in the sensor data and providing mutually exclusive binarised parameters for the processing.

**4.** A method according to any of the preceding claims, wherein
the aspects included within the collections vary to give two or more different collection types, the sequence of collections preferably alternating between two different collection types, and preferably wherein
the collection type changes at each new time instant, or at every second time instant.

**5.** A method according to any of the preceding claims, wherein
analysing the state machine includes storing all the states detected over the period of time and the transitions between them, and preferably includes calculation of the total time spent in each state and the probability of transition to other detected states.

**6.** A method according to any of the preceding claims, wherein
the state machine is displayed based on a grid of potential states, with N axes, an axis for each of N aspects displayed, the states detected being represented on the grid, transitions between states preferably also being represented; and/ wherein
the state machine is displayed based on a grid of potential states and time, with N+1 axes, an axis for each of N aspects displayed and an axis for time, the states detected and their timing being represented.

**7.** A method according to any of the preceding claims, wherein
each state detected in the state machine or each potential state of a system model is compared to a database of medical rules, and preferably wherein a warning is provided if the state does not conform to the rules, and the database of medical rules is related to symptoms of one or more diseases or medical conditions.

**8.** A method according to claim 7, wherein
the result of the comparison is a classification of each state as in violation of, in line with, or not relevant to one or more of the rules, and wherein each state is marked accordingly.

**9.** A method according to claim 8, wherein
the marking is provided on a representation of the states, with one marking for any state in violation of on or more of the rules, another marking for any state in line with one or more of the rules and a third marking for any state not relevant to one or more of the rules.

**10.** A method according to any of claims 7 to 9, wherein
the states in a system model are divided into a set of one or more clusters, including any of: a problem cluster for all the states which are in violation of one or more of the rules; a must-have cluster for all the states which are in line with one or more of the rules; and a don't care cluster for all the states which are not relevant to one or more of the rules; and each detected state is automatically allocated to a cluster of the system model; or wherein each detected state is compared with the database of medical rules and the detected states are divided into a set of one or more clusters, including any of: a problem cluster for all the states which are in violation of one or more of the rules; a must-have cluster for all the states which are in line with one or more of the rules; and a don't care cluster for all the states which are not relevant to one or more of the rules.

**11.** A method according to claim 10, wherein
a set of clusters is provided for each rule of a grouping of rules corresponding to the symptoms of a disease or medical condition, and two or more sets of clusters are combined to form a set of overall clusters taking into account two or more symptoms.

**12.** A method according to claim 10 or 11, wherein the states in each cluster are distinguished by colour coding.

**13.** A state machine apparatus arranged to analyse sensor data in the form of parameters describing the condition of a subject, the apparatus comprising:

a processor operable to process the sensor data to provide, at each of a plurality of time instants, a collection of two or more of the parameters, each of which parameters describes a different aspect of the subject's condition, the parameters together representing a state of the subject;
a sequencer operable to put together successive collections to form a sequence of states; and
an analyser operable to analyse the sequence to create a state machine representing the states of the subject detected over a period of time comprising the plurality of time instants.

**14.** A sensing system operable to analyse sensor data in the form of parameters describing the condition of a subject, the system comprising :

an apparatus according to claim 21; and
one or more sensors which are operable to gather information which can be used to describe the condition of a subject.

**15.** A computer program which when executed on a computing apparatus causes it to carry out the method of any of claims 1 to 20, or which when downloaded onto a computing apparatus causes it to become the state machine apparatus of claim 21.

Raw sensor data
over a period of time

S10 ⟍ | Processing |

Collection of parameters (state)

S20 ⟍ | Sequence formed |

Sequence of states

S30 ⟍ | Analysing |

State machine over
a period of time

FIG. 1

FIG. 2

Parallel Raw Sensory Data
from Environment with 0 and 1 values

Symptom Related Sensor Data 1
Symptom Related Sensor Data 2
Symptom Related Sensor Data 3
Symptom Related Sensor Data 4
Symptom Related Sensor Data 5
Symptom Related Sensor Data 6
Symptom Related Sensor Data 7
Symptom Related Sensor Data 8
Symptom Related Sensor Data 9
Symptom Related Sensor Data 10
Symptom Related Sensor Data 11
Symptom Related Sensor Data 12
Symptom Related Sensor Data 13
Symptom Related Sensor Data 14
Symptom Related Sensor Data 15
Symptom Related Sensor Data 16
Symptom Related Sensor Data 17
Symptom Related Sensor Data 18
Symptom Related Sensor Data 19
Symptom Related Sensor Data 20
Symptom Related Sensor Data 21
Symptom Related Sensor Data 22
Symptom Related Sensor Data 23

21

Grouping
and
Classification

Symptom Related Sensor Data 1 S1
Symptom Related Sensor Data 2 S2    } Group 1
Symptom Related Sensor Data 3 S3

Symptom Related Sensor Data 4 S4
Symptom Related Sensor Data 5 S5
Symptom Related Sensor Data 6 S6
Symptom Related Sensor Data 7 S7
Symptom Related Sensor Data 8 S8
Symptom Related Sensor Data 9 S9       Group 2
Symptom Related Sensor Data 10 S10
Symptom Related Sensor Data 11 S11
Symptom Related Sensor Data 12 S12
Symptom Related Sensor Data 13 S 13

Symptom Related Sensor Data 14 S14
Symptom Related Sensor Data 15 S15
Symptom Related Sensor Data 16 S16
Symptom Related Sensor Data 17 S17
Symptom Related Sensor Data 18 S18    Group 3
Symptom Related Sensor Data 19 S19
Symptom Related Sensor Data 20 S20
Symptom Related Sensor Data 21 S21
Symptom Related Sensor Data 22 S22
Symptom Related Sensor Data 23 S23

FIG. 3 Grouping and classification function to create the three main data stream of whereabouts, activities and gait

Symptom Related Sensor Data 1 S1  
Symptom Related Sensor Data 2 S2  }Group 1  
Symptom Related Sensor Data 3 S3  

Symptom Related Sensor Data 4 S4  
Symptom Related Sensor Data 5 S5  
Symptom Related Sensor Data 6 S6  
Symptom Related Sensor Data 7 S7  
Symptom Related Sensor Data 8 S8  
Symptom Related Sensor Data 9 S9  } Group 2  
Symptom Related Sensor Data 10 S10  
Symptom Related Sensor Data 11 S11  
Symptom Related Sensor Data 12 S12  
Symptom Related Sensor Data 13 S 13  

Symptom Related Sensor Data 14 S14  } Group 3  
Symptom Related Sensor Data 15 S15  
Symptom Related Sensor Data 16 S16  
Symptom Related Sensor Data 17 S17  
Symptom Related Sensor Data 18 S18  
Symptom Related Sensor Data 19 S19  
Symptom Related Sensor Data 20 S20  
Symptom Related Sensor Data 21 S21  
Symptom Related Sensor Data 22 S22  
Symptom Related Sensor Data 23 S23  

22

Sampling

70

Sequence of Two
Parameter States

| S1-S14 | S4-S15 | S3-S17 | ·······

Sampling
Time

FIG. 4 Sampling function to create the two parameter states

EP 2 528 016 A1

FIG. 5 Sampling function to create the two parameter states

FIG. 6 Buffering and state machine formation function to create the final state machine

FIG. 7

Overall architecture for creation of human state machine based on the raw sensor data

FIG. 7 (continued)

FIG. 8 How to create the clusters

Parallel Raw Sensory Data
from Environment with 0 and 1 values

Normal Walking (NW)
Walking with Difficulty (DW)
Fall (FW)
Living Room (LV)
Office Room (OR)
Bath Room (BT)
WC (WC)
Kitchen (KI)
Bedroom (BR)
Balcony (BA)
Lounge (LO)
Outside Home (OH)

Exercise A: Living Room (EA)
Exercise B: Office Room (EB)
Exercise C: Kitchen (EC)
Exercise M: (EM) Medicine
Exercise V: (EV ) Vitamins
Exercise F: Internet Surfing (IS)
Exercise G: Watching TV (TV)
Exercise H: Communications (CO)
Sleep (SL)
Any Other Activity (AO)

21
Grouping and Classification

Normal Walking (NW)
Walking with Difficulty (DW)
Fall (FW)  } Gait

Living Room (LV)
Office Room (OR)
Bath Room (BT)
WC (WC)
Kitchen (KI)
Bedroom (BR)
Balcony (BA)
Lounge (LO)
Outside Home (OH)  } Where abouts

Exercise A: Living Room (EA)
Exercise B: Office Room (EB)
Exercise C: Kitchen (EC)
Exercise M: (EM) Medicine
Exercise V: (EV ) Vitamins
Exercise F: Internet Surfing (IS)
Exercise G: Watching TV (TV)
Exercise H: Communications (CO)
Sleep (SL)
Any Other Activity (AO)  } Activities

FIG. 9 Specific examples of grouping and classification function to create the three main data stream of whereabouts, activities and gait

EP 2 528 016 A1

Normal Walking (NW)
Walking with Difficulty (DW)     } Gait
Fall (FW)

Living Room (LV)
Office Room (OR)
Bath Room (BT)
WC (WC)
Kitchen (KI)                     } Where
Bedroom (BR)                       about
Balcony (BA)
Lounge (LO)
Outside Home (OH)

Exercise A: Living Room (EA)
Exercise B: Office Room (EB)
Exercise C: Kitchen (EC)
Exercise M: (EM) Medicine
Exercise V: (EV ) Vitamins
Exercise F: Internet Surfing (IS)     } Activities
Exercise G: Watching TV (TV)
Exercise H: Communications (CO)
Sleep (SL)
Any Other Activity (AO)

Sampling

Sequence of Two
Parameter States

| LV-NW | BA-DW | OH-EA | · · · · · · · ·

Sampling
Time

FIG. 10 Specific examples of sampling function to create the two parameter states

FIG. 11 Specific examples of sampling function to create the two parameter states

Final State Machine

Normal Person's State Machine based on the Test Data Set Created based on the Symptoms from the Johns Hopkins Research Findings:

Sequence of Two Parameter States

| NW-LV | BA-DW | LV-EA | ⋯⋯ | OH-EA |

Sampling Time

Duration and Start of Analysis

39 — Data Buffer

State Machine Formation Function

40

S1 S14   S4 S15   S1 S16   S13 S17

S2 S19   S2 S18

S7 S19   S10 S18

S1 S14   S9 S19

S11 S18   S3 S22

S2 S14   S11 S20

S7 S19   S7 S17   S5 S20   S8 S21   S8 S23

The way human subject walks:

Normal Walking (NW)
Walking with Difficulty (DW)
Fall (FW)

Where human subject is:
Living Room (LV)
Office Room (OR)
Bath Room (BT)
WC (WC)
Kitchen (KI)
Bedroom (BR)
Balcony (BA)
Lounge (LO)
Outside Home (OH)

What human subject is doing:
Exercise A: Living Room (EA)
Exercise B: Office Room (EB)
Exercise C: Kitchen (EC)
Exercise M: (EM) Medicine
Exercise V: (EV ) Vitamins
Exercise F: Internet Surfing (IS)
Exercise G: Watching TV (TV)
Exercise H: Communications(CO)
Sleep (SL)
Any Other Activity (AO)

80

FIG. 12 Specific examples of buffering and state machine formation function to create the final state machine

Passive Sensor Network in the Living Environment                                    MATLAB Program AC_1.m

Raw Sensory
Data from Environment

Normal Walking (NW)
Walking with Difficulty (DW)
Fall (FW)
Living Room (LV)
Office Room (OR)
Bath Room (BT)
WC (WC)
Kitchen (KI)
Bedroom (BR)
Balcony (BA)
Lounge (LO)
Outside Home (OH)
Exercise A: Living Room (EA)
Exercise B: Office Room (EB)
Exercise C: Kitchen (EC)
Exercise M: (EM) Medicine
Exercise V: (EV ) Vitamins
Exercise F: Internet Surfing (IS)
Exercise G: Watching TV (TV)
Exercise H: Communications (CO)
Sleep (SL)
Any Other Activity (AO)

Grouping
and
Classification

Normal Walking (NW)
Walking with Difficulty (DW)
Fall (FW)

Living Room (LV)
Office Room (OR)
Bath Room (BT)
WC (WC)
Kitchen (KI)
Bedroom (BR)
Balcony (BA)
Lounge (LO)
Outside Home (OH)

Exercise A: Living Room (EA)
Exercise B: Office Room (EB)
Exercise C: Kitchen (EC)
Exercise M: (EM) Medicine
Exercise V: (EV ) Vitamins
Exercise F: Internet Surfing (IS)
Exercise G: Watching TV (TV)
Exercise H: Communications (CO)

Sleep (SL)
Any Other Activity (AO)

Overall architecture for creation of human state machine based on the raw sensory data
related to syndromes of the Alzheimer's disease with MATLAB implementation

FIG. 13

FIG. 13 (continued)

MATLAB Program
Determine_StateMachine_Daily.m

Final State Machine

Normal Person's State Machine based on the Test Data Set Created
based on the Symptoms form the Johns Hopkins Research Findings:

Duration and Start
of Analysis

S4 S15   S1 S16      S13 S17

S1 S14

S2 S19

S7 S19

S1 S14

S11 S18

S2 S14

S2 S18

S10 S18

S9 S19

S3 S22

S11 S20

S5 S20   S8 S21

S7 S19   S7 S17   S8 S23

State Machine Formation Function

Data Buffer

The way human subject walks:

Normal Walking (NW)
Walking with Difficulty (DW)
Fall (FW)

Where human subject is:
Living Room (LV)
Office Room (OR)
Bath Room (BT)
WC (WC)
Kitchen (KI)
Bedroom (BR)
Balcony (BA)
Lounge (LO)
Outside Home (OH)

What human subject is doing:
Exercise A: Living Room (EA)
Exercise B: Office Room (EB)
Exercise C: Kitchen (EC)
Exercise M: (EM) Medicine
Exercise V: (EV ) Vitamins
Exercise F: Internet Surfing (IS)
Exercise G: Watching TV (TV)
Exercise H: Communications(CO)
Sleep (SL)
Any Other Activity (AO)

MATLAB Program
New_Analyse_Draw_Score.m

FIG. 13 (continued)

FIG. 14 Outcome of MATLAB platform: One week of test data for a normal person

FIG. 15 Outcome of MATLAB platform: One week of test data for the Alzheimer's patient

Human State Machine Day 8

FIG. 17

Human State Machine Day 1

FIG. 16

FIG. 18 Outcome of MATLAB platform: weekly Human State Machine, normal person, week 1

FIG. 19 Outcome of MATLAB platform: weekly Human State Machine, person with Alzheimer's disease syndromes, week 2

FIG. 20 Basic Grid of States for forming the 2D Colour-Coded Human State Machine

FIG. 21 Walking Symptom based Full Cluster: Dark shading, Difficulty Walking (DW) or Fall while Walking (FW), Light shading, Normal Walk, (NW)

FIG. 22 Memory Loss Symptom and Exercise based Full Cluster: Dark shading, Exercise in a Wrong Location, Light shading, Right Exercise in the Right Location and Right Time

FIG. 23 Memory and Walking Status Full Combined Clusters:Dark shading, Trouble Hot Spot, Light shading, Must Have Areas

FIG. 24 A representation of the memory exercise related rules for HSM when the critical timing of the activities matters in addition to the location, light shading, Exercise in a wrong location or wrong time, light point, Right exercise in the right location and right time, dark shading Don't Care: All the Possibilities Included

EP 2 528 016 A1

$$Sypmt=\{Sympt_l\}, \quad l=1...L$$
Symptom A index

Symptom
Database

Rules Related to the Symptoms
including the states and activities
or transitions of the interest or
proffered activity time

Sequence of Two
Parameter States
in Time

$$S=\{S_k\}=\{Loc_m,Act_n,\{Sin_s\},\{Sout_d\},\{Tstart_i\},\{Tspent_i\},m=1...$$
$$M,n=1...N,s=1...S,d=1...D,i=1...I,k=1...M.N$$

Sampling
&
Classifications

| NW-LV | BA-DW | OH-EA |

Does the location
and activity violate
the roles?

No

Yes

Colour Code Green

Irrelevant

Colour Code Red

Colour Code Black

Capture and
Expand the Cluster

Capture and
Expand the Cluster

Capture and
Expand the Cluster

Green Cluster
of Symptom A

Black Cluster
Don't Care

Red Cluster
of Symptom A

Result: $C_A=\{\{S_t\},\{S_q\},\{R_h\}\}, \quad t=1...T,q=1...Q,h=1...H$

FIG. 25 How to Create the Clusters

EP 2 528 016 A1

FIG. 26 How to Group the Clusters

40

FIG. 27

FIG. 28

FIG. 29

FIG. 30

FIG. 31

EP 2 528 016 A1

FIG. 32

FIG. 33

FIG. 34

FIG. 35

Europäisches
Patentamt

European
Patent Office

Office européen
des brevets

**EUROPEAN SEARCH REPORT**

Application Number

EP 11 16 7737

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 2 018 825 A1 (ECOLE POLYTECH [CH]) 28 January 2009 (2009-01-28) * abstract * ----- | 1-15 | INV. G06K9/00 A61B5/11 |
| | | | TECHNICAL FIELDS SEARCHED (IPC) |
| | | | A61B G06K G06F |

The present search report has been drawn up for all claims

| Place of search | Date of completion of the search | Examiner |
|---|---|---|
| The Hague | 7 December 2011 | Sonius, Michiel |

EPO FORM 1503 03.82 (P04C01)

## ANNEX TO THE EUROPEAN SEARCH REPORT
## ON EUROPEAN PATENT APPLICATION NO.

EP 11 16 7737

This annex lists the patent family members relating to the patent documents cited in the above-mentioned European search report.
The members are as contained in the European Patent Office EDP file on
The European Patent Office is in no way liable for these particulars which are merely given for the purpose of information.

07-12-2011

| Patent document cited in search report | Publication date | Patent family member(s) | Publication date |
|---|---|---|---|
| EP 2018825 A1 | 28-01-2009 | NONE | |

EPO FORM P0459

For more details about this annex : see Official Journal of the European Patent Office, No. 12/82